# EUROPÄISCHE PATENTSCHRIFT

(11) **EP 1 742 926 B1**
(45) Veröffentlichungstag und Bekanntmachung des Hinweises auf die Patenterteilung: **08.08.2007**
(21) Anmeldenummer: 05742880.7
(22) Anmeldetag: 19.04.2005
(51) Int. Cl.: C07D 257/02, A61K 51/04, A61K 49/08

(54) **TRIMERE MAKROCYCLISCH SUBSTITUIERTE HALOGEN-BENZOLDERIVATE**
MACROCYCLE-SUBSTITUTED TRIMER HALOGEN-BENZOL DERIVATIVES
DERIVES TRIMERES DE BENZENE HALOGENE SUBSTITUES PAR DES MACROCYCLES

(30) Priorität: 05.05.2004 DE 102004023093
(43) Veröffentlichungstag der Anmeldung: 17.01.2007
(73) Patentinhaber: Bayer Schering Pharma Aktiengesellschaft, 13353 Berlin (DE)
(72) Erfinder: HARTO, Juan R., E-28005 Madrid (ES); MARTIN, José L., E-8220 Majadahonda (Madrid) (ES); PLATZEK, Johannes, 12621 Berlin (DE); SCHIRMER, Heiko, 12161 Berlin (DE); WEINMANN, Hanns-Joachim, 14129 Berlin (DE); CARRETERO, Jose, E-28030 Madrid (ES)
(86) Internationale Anmeldenummer: PCT/EP2005/004319
(87) Internationale Veröffentlichungsnummer: WO 2005/108379

(56) Entgegenhaltungen:
- WO-A-20/04074267
- US-A- 5 660 814

## Beschreibung

Die Erfindung betrifft die in den Patentansprüchen gekennzeichneten Gegenstände: neue trimere makrocyclisch substituierte Trijod- und Tribrombenzolderivate, deren Herstellung und Verwendung als Kontrastmittel in der Röntgen- und MRT-Diagnostik.

Während des letzten Jahrzehntes wurden in der bildgebenden Diagnostik beeindruckende Fortschritte erzielt. Die Bildgebungstechniken wie DAS, CT und MRT haben sich zu den normalen und unverzichtbaren Werkzeugen in der Diagnostik und interventionellen Radiologie entwickelt und bieten heute eine räumliche Auflösung von weniger als 1 mm. Die Anwendungsmöglichkeiten dieser Techniken werden weiterhin entscheidend durch den Einsatz von Kontrastmitteln erhöht. Diese heutige weite Verbreitung und Akzeptanz der Kontrastmittel in der Röntgendiagnostik ist auf die Einführung von nichtionischen monomeren Triiodaromaten in den 80er Jahren, sowie die in den 90er Jahren eingeführten isoosmolaren dimeren lodaromaten zurückzuführen. Durch diese beiden Verbindungsklassen wurde die Frequenz von kontrastmittelinduzierten Nebenwirkungen auf 2 - 4% reduziert (Bush W.H., Swanson D.P. : Acute reactions to intravascular contrast media: Types, risk factors, recognition and specific treatment. AJR 157, 1153-1161, 1991. Rydberg J., Charles J., Aspelin P.: Frequency of late allergy-like adverse reactions following injection of intravascular non-ionic contrast media. Acta Radiológica 39, 219-222, 1998). Die Anwendung der Kontrastmittel in Verbindung mit den modernen Bildgebungstechniken reicht heute von der Detektion von Tumoren, zur hochauflösenden Gefäßdarstellung, bis hin zur quantitativen Bestimmung von physiologischen Faktoren wie Permeabilität oder Perfusion von Organen. Maßgeblich für den Kontrast und die Nachweisempfindlichkeit ist die Konzentration des Röntgenkontrastmittels (hier des lod Atoms). Trotz Weiterentwicklung der Technik konnte die für eine medizinische Diagnose notwendige Konzentration bzw. die zu applizierende Dosis nicht reduziert werden. So werden in einer klassischen CT Untersuchung 100 g Substanz oder mehr pro Patient injiziert.

Obgleich die Verträglichkeit der Röntgenkontrastmittel durch die Einführung der nichtionischen Triiodbenzole verbessert worden ist, sind die Nebenwirkungen noch immer hoch. Aufgrund der sehr hohen Untersuchungszahlen von mehreren Millionen pro Jahr in der Röntgendiagnostik sind somit zehntausende Patienten betroffen. Diese kontrastmittelinduzierten Nebenwirkungen reichen von leichten Reaktionen wie Nausea, Schwindelgefühl, Erbrechen, Urticaria bis hin zu schweren Reaktionen wie Bronchospasmus, Nierenversagen bis zu Reaktionen wie Schock oder sogar Tod. Glücklicherweise sind diese schweren Fälle sehr selten und werden mit einer Häufigkeit von nur 1/200000 beobachtet (Morcos S.K., Thomsen H.S.: Adverse reactions to iodinated contrast media. Eur Radiol 11, 1267-1275, 2001).

Die Häufigkeit dieser auch als pseudoallergischen kontrastmittelinduzierten beobachteten Nebenwirkungen wird aber ca. um den Faktor 3 bei atopischen Patienten, und um den Faktor 5 bei Patienten mit einer Vorgeschichte kontrastmittelinduzierter Nebenwirkungen erhöht. Asthma erhöht das Risiko schwerer kontrastmittel-induzierter Nebenwirkungen um den Faktor 6 bei nichtionischen Kontrastmitteln (Thomsen H.S., Morcos S.K.: Radiographic contrast media. BJU 86 (Suppl1), 1-10, 2000. Thomsen H.S., Dorph S.: High-osmolar and low-osmolar contrast media. An update on frequency of adverse drug reactions. Acta Radiol 34, 205-209, 1993. Katayama H, Yamaguchi K., Kozuka T., Takashima T., Seez P., Matsuura K.: Adverse reactions to ionic and nonionic contrast media. Radiology 175, 621-628, 1990. Thomsen H.S., Bush Jr W.H.: Adverse effects on contrast media. Incidence, prevention and management. Drug Safety 19: 313-324, 1998). In diesen Situationen verwenden die Untersucher für die Röntgendiagnostik in letzter Zeit immer häufiger nichtiodhaltige Gd-Chelate anstelle der klassischen Triiodaromaten in der Computertomographie aber auch in der interventionellen Radiology sowie der DSA (Gierada D.S., Bae K.T.: Gadolinium as CT contrast agent: Assessment in a porcine model. Radiology 210, 829-834, 1999. Spinosa D.J., Matsumoto A.H., Hagspiel K.D., Angle J.F., Hartwell G.D.:Gadolinium-based contrast agents in angiography and interventional radiology. AJR 173; 1403-1409, 1999. Spinosa D.J., Kaufmann J.A., Hartwell G.D. : Gadolinium chelates in angiography and interventional radiology: A useful alternative to iodinated contrast media for angiography. Radiology 223, 319-325, 2002). Dies ist zum einen durch die sehr gute Verträglichkeit der in der MRT verwendeten Metallchelate begründet, aber auch durch die bekannte Tatsache, dass Lanthanide ebenfalls röntgendicht sind. Gadolinium und andere Lanthaniden zeigen gegenüber lod insbesondere bei höheren Spannungen/Energien der Röntgenstrahlung eine größere Absorption als lod, so dass sie prinzipiell als kontrastgebende Elemente für die Röntgendiagnostik geeignet sind (Schmitz S., Wagner S., Schuhmann-Giampieri G., Wolf K.J.: Evaluation of gadobutrol in an rabbit model as a new lanthanide contrast agent for computer tomography. Invest. Radiol. 30(11): 644-649, 1995).

Die genannten ursprünglich in der MRT eingesetzten Gd-haltigen Chelatverbindungen sind ebenfalls gut wasserlöslich und zeichnen sich durch eine exzellente Verträglichkeit aus. Gegenüber den iodhaltigen/nichtionischen Kontrastmitteln ist die Rate leichter pseudoallergischer Reaktionen stark verringert, die Rate fataler Reaktionen ist extrem selten und wird mit 1/1000000 angegeben (Runge V.M.: Safety of approved MR contrast media for intravenous injection. J. Magn Reson Imaging 12, 205-213, 2000). Pseudoallergische Reaktionen sind im Gegensatz zu anderen kontrastmittelinduzierten Nebenwirkungen, wie z.B. die Nierenverträglichkeit, eher unabhängig von der verabreichten Dosis. Auch kleinste Dosierungen können demnach schon eine pseudoallergische Reaktion auslösen.

Gewünscht sind Substanzen, die die Vorteile beider chemisch total unterschiedlicher Verbindungsklassen vereinen.

Für eine geringe Unverträglichkeitsrate spricht die außerordentlich hohe Hydrophilie der Metallchelate. lodaromate weisen ein um den Faktor 100-200 höhere Lipophilie (größerer Verteilungskoeffizient zwischen Butanol/Wasser) als Metallchelate auf.

Aufgrund der geringen Substanzkonzentration und des geringen spezifischen Anteils des bildgebenden Metalls am Gesamtmolekül sind die bisher bekannten Metallchelate für die Röntgendiagnostik nicht optimal (Albrecht T., Dawson P.: Gadolinium-DTPA as X-ray contrast medium in clinical studies. BJR 73, 878-882, 2000). Neuere Ansätze zur Lösung dieses Problems beschreiben die Herstellung von Metallkomplexkonjugaten, in denen an einen offenkettigen oder makrocyclischen Metallkomplex Triiodaromaten kovalent gebunden sind (US 5,324,503, US 5,403,576, WO 93/16375, WO 00/75141, WO 97/01359, WO 00/71526, insbesondere die US 5,660,814). Wegen ihrer geringen Hydrophilie und hohen Viskosität sind diese jedoch nicht in ausreichender Konzentration und vertretbaren Volumina zu applizieren.
Die im nächstliegendem Stand der Technik aus der US 5,660,814 offenbarten Verbindungen 3 aus Example 3 und 4 aus Example 4 sind im Vergleich zu den erfindungsgemäßen Verbindungen
1) ionisch und haben somit eine um Faktor 2 höhere Osmolalität im Vergleich zu den erfindungsgemäßen neutralen Verbindungen, was besonders negativ bei hohe Dosen ist,
2) diese sind wesentlich liphophiler als die erfindungsgemäßen Verbindungen (Anmerkung in Spalte 5/Zeile 29 der US 5 660 814, die Verbindungen aus dem Stand der Technik sind als Leberkontrastmittel verwendbar),
3) die Substanzen 3 und 4 der US 5 660 814 sind wesentlich toxischer als die erfindungsgemäßen Verbindungen (siehe LD₅₀ sowie Verteilungskoefficient) und
4) die Relaxivity für die Bildgebung im MR ist somit schlechter.

Ziel ist es Verbindungen herzustellen, die eine ausreichende Hydrophilie - vergleichbar derjenigen von Gd-Chelaten - besitzen und zusätzlich eine hohe Konzentration von kontrastgebenden Elementen aufweisen. Werte, die deutlich höher sind als die bei etwa 25 % (g/g) liegenden Metallchelate, wären wünschenswert. Bei einer höheren Konzentration muss weiterhin eine sehr gute Wasserlöslichkeit gegeben sein. Die hochkonzentrierten Lösungen müssen neben ihren guten pharmakologischen Eigenschaften ebenfalls eine praktikable Viskosität und einen niedrigen osmotischen Druck aufzeigen.

Diese Aufgabe wird durch die vorliegende Erfindung gelöst.

Die erfindungsgemäßen Metallkomplexe der allgemeinen Formel I 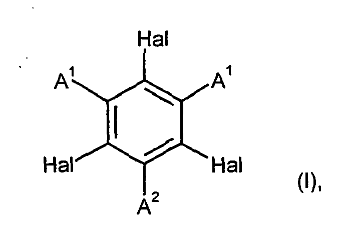 worin
Hal für Brom oder Jod,
A¹ für den Rest
-CONR¹-(CH₂)ₙ-NR²-(CO-CHZ¹-NH)ₘ-CO-CHZ²-K,
A² für den Rest-NR¹-CO-CHZ²-K stehen,
in denen R¹ und R² unabhängig ein Wasserstoffatom, eine C₁-C₂-Alkylgruppe oder eine Monohydroxy-C₁-C₂-Alkylgruppe,
Z¹ und Z² unabhängig voneinander ein Wasserstoffatom oder eine Methylgruppe,
n die Ziffern 2-4,
m die Ziffern 0 oder 1 und
K für einen Makrocyclus der Formel I_{A} 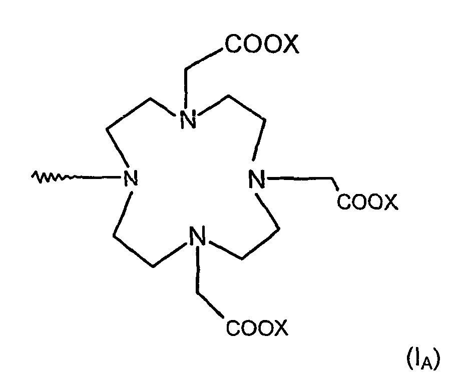 mit X in der Bedeutung eines Wasserstoffatoms oder eines Metallionenäquivalents der Ordnungszahlen 20-29,39, 42, 44 oder 57-83 stehen, mit den Maßgaben, dass mindestens zwei X für Metallionenäquivalente stehen und gegebenenfalls vorhandene freie Carboxygruppen gegebenenfalls als Salze organischer und/oder anorganischer Basen oder Aminosäuren oder Aminosäureamide vorliegen, zeigen eine sehr gute Löslichkeit und einen Verteilungskoeffizienten, der mit dem von Gd-Chelaten vergleichbar ist. Weiterhin weisen die neuen Verbindungen einen hohen spezifischen Gehalt an kontrastgebenden Elementen, eine niedrige Viskosität und Osmolalität, und damit gute Toleranz/Verträglichkeit auf, so dass sie hervorragend als Kontrastmittel für die Röntgen- und MR-Bildgebung geeignet sind.

Bevorzugt stehen Hal für lod, R¹ und R² für Wasserstoff und die Methylgruppe,
m für die Ziffer 0 und n für die Ziffer 2.

Beispielhaft genannte Reste A¹ sind:

-CONH(CH₂)_{2;3}NHCOCH₂NHCOCH(CH₃)-K,

-CONH(CH₂)_{2;3}NHCOCH₂NHCOCH₂-K,

-CONH(CH₂)_{2;3}NHCOCH₂-K,

-CONH(CH₂)_{2;3}NHCOCH(CH₃)-K,

-CON(CH₂CH₂OH(CH₂)₂NHCOCH₂-K

Beispielhaft genannte Reste A² sind:

-NHCOCH(CH₃)-K,

-NHCOCH₂-K,

-N(CH₃)COCH₂-K,

-N(CH₃)COCH(CH)₃-K,

-N(CH₂CH₂OH)COCH₂-K,

-N(CH₂CH₂OH)COCH(CH₃)-K.

Die erfindungsgemäßen Verbindungen der allgemeinen Formel I lassen sich nach dem Fachmann bekannten Verfahren herstellen, indem man einen Trijod- oder Tribromaromaten der allgemeinen Formel II 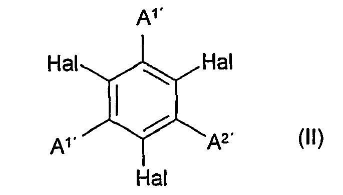 in an sich bekannter Weise mit einem Makrocyclus der allgemeinen Formel III 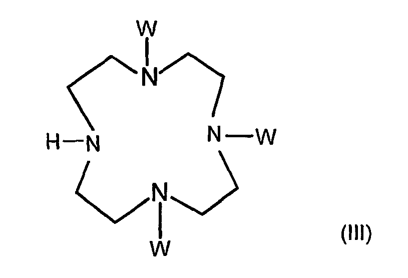 worin

W für eine Schutzgruppe oder eine -CH₂COOX'-gruppe mit X' in der Bedeutung von X oder einer Schutzgruppe und A^{1'} in der Bedeutung von -CO-NR¹-(CH₂)ₙ-NR²-(CO-CHZ¹-NH)ₘ-CO-CHZ²-Hal' und A^{2'} für-NR¹-CO-CHZ²-Hal' mit Hal' in der Bedeutung von Chlor oder Brom, stehen, umsetzt und anschließend gegebenenfalls die Schutzgruppe W entfernt und die Reste CH₂COOX in an sich bekannter Weise einführt bzw. die für X' gegebenenfalls stehende Schutzgruppe entfernt und anschließend in an sich bekannter Weise mit einem Metalloxid oder Metallsalz eines Elements der Ordnungszahlen 20-29, 39, 42, 44 oder 57-83 umsetzt.

Als Aminoschutzgruppen W seien die dem Fachmann geläufigen Benzyloxycarbonyl-, tertiär-Butoxycarbonyl-, Trifluoracetyl-, Fluorenylmethoxycarbonyl-, Benzyl-, Formyl-, 4-Methoxybenzyl-, 2,2,2-Trichlorethoxycarbonyl-, Phthaloyl-, 1,2-Oxazolin-, Tosyl-, Dithiasuccinoyl-, Allyloxycabonyl-, Sulfat-, Pent-4-encarbonyl-, 2-Chloracetoxymethyl (bzw.-ethyl) benzoyl-, Tetrachlorphthaloyl-, Alkyloxycarbonylgruppen genannt [Th. W. Greene, P.G.M. Wuts, Protective Groups in Organic Syntheses, 2nd ed, John Wiley and Sons (1991), S. 309 - 385; E. Meinjohanns et al, J. Chem. Soc. Pekin Trans 1, 1995, 405; U. Ellensik et al, Carbohydrate Research 280, 1996, 251; R. Madsen et al, J. Org. Chem. 60, 1995, 7920; R.R. Schmidt, Tetrahedron Letters 1995, 5343].

Die Abspaltung der Schutzgruppen erfolgt nach den dem Fachmann bekannten Verfahren (s. z.B. E. Wünsch, Methoden der Org. Chemie, Houben-Weyl, Bd XV/1, 4. Auflage 1974, S. 315), beispielsweise durch Hydrolyse, Hydrogenolyse, alkalische Verseifung der Ester mit Alkali in wäßrig-alkoholischer Lösung bei Temperaturen von 0 °C bis 50 °C, saure Verseifung mit Mineralsäuren oder im Fall von Boc-Gruppen mit Hilfe von Trifluoressigsäure.

Falls X für eine Säureschutzgruppe steht, kommen niedere Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis-(p-Nitrophenyl)-methylgruppe, sowie Trialkylsilylgruppen in Frage.

Bevorzugt sind die t-Butyl- und die Benzylgruppe.

Die Abspaltung der Schutzgruppen erfolgt nach den dem Fachmann bekannten Verfahren (s. z.B. E.Wünsch, Methoden der Org. Chemie, Houben-Weyl, Bd XV/1, 4. Auflage 1974, S. 315), beispielsweise durch Hydrolyse, Hydrogenolyse, alkalische Verseifung der Ester in wäßrig-alkoholischer Lösung bei Temperaturen von 0 °C bis 50 °C, saure Verseifung mit Mineralsäuren oder im Fall von tert.-Butylestern mit Hilfe von Trifluoressigsäure (Protective Groups in Organic Synthesis, 2nd Edition, T.W. Greene und P.G.M.Wuts, John Wiley and Sons Inc., New York, 1991).

Die Einführung der gewünschten Metallionen kann in der Weise erfolgen, wie sie in den Patentschriften EP 71564, EP 130934 und DE-OS 34 01 052 offenbart worden ist. Dazu wird das Metalloxid oder ein Metallsalz (beispielsweise ein Chlorid, Nitrat, Acetat, Carbonat oder Sulfat) des gewünschten Elements in Wasser und/oder einem niederen Alkohol (wie Methanol, Ethanol oder Isopropanol) gelöst oder suspendiert und mit der Lösung oder Suspension der äquivalenten Menge des Komplexbildners umgesetzt.
Die Neutralisation eventuell noch vorhandener freier Carboxygruppen erfolgt mit Hilfe anorganischer Basen (z.B. Hydroxyden, Carbonaten oder Bicarbonaten) von z.B. Natrium, Kalium, Lithium, Magnesium oder Calcium und/oder organischer Basen wie unter anderem primärer, sekundärer und tertiärer Amine, wie z.B. Ethanolamin, Morpholin, Glucamin, N-Methyl- und N,N-Dimethylglucamin, sowie basischer Aminosäuren, wie z.B. Lysin, Arginin und Ornithin oder von Amiden ursprüngliche neutraler oder saurer Aminosäuren.

Zur Herstellung der neutralen Komplexverbindungen kann man beispielsweise in sauren Komplexsalzen in wässriger Lösung oder Suspension soviel der gewünschten Base zusetzen, dass der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie z.B. niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Die Reinigung der so erhaltenen Komplexe erfolgt, gegebenenfalls nach Einstellung des pH-Wertes durch Zusatz einer Säure oder Base auf pH 6 bis 8, bevorzugt ca. 7, vorzugsweise durch Ultrafiltration mit Membranen geeigneter Porengröße (z.B. Amicon^{®}YM1, Amicon^{®}YM3), Gelfiltration an z.B. geeigneten Sephadex^{®}-Gelen oder durch HPLC an Kieselgel oder reversephase Material.

Eine Reinigung kann auch durch Kristallisation aus Lösungsmitteln wie Methanol, Ethanol, i-Propanol, Aceton oder deren Mischungen mit Wasser erfolgen.

Im Falle von neutralen Komplexverbindungen ist es häufig von Vorteil, die oligomeren Komplexe über einen Anionenaustauscher, beispielsweise IRA 67 (OH--Form) und gegebenenfalls zusätzlich über einen Kationenaustauscher, beispielsweise IRC 50 (H⁺-Form) zur Abtrennung ionischer Komponenten zu geben

Die Herstellung der erfindungsgemäßen Verbindungen der allgemeinen Formel 1 kann wie oben angegeben erfolgen:

Die Umsetzung von Trijod- oder Tribromaromaten der allgemeinen Formel ll mit Verbindungen der allgemeinen Formel lll erfolgt nach den dem Fachmann bekannten Verfahren der Alkylierung von N-haltigen Macrocyclen. Hierbei werden Verbindungen der allgemeinen Formel II, wobei A^{1'} für -CO-NR¹-(CH₂)ₙ-NR²-(CO-CHZ¹-NH)ₘ-CO-CHZ²Hal'und A^{2'} für -NR¹-CO-CHZ²Hal', mit Hal' in der Bedeutung von Chlor oder Brom, stehen mit dem freien Amin von III alkyliert, indem man in aprotischen Lösungsmitteln wie Acetonitril, Methylenchlorid, Chloroform, DMF, DMA, THF, Dioxan oder Toloul bei Temperaturen von 0° - 80° C gegebenenfals unter Zusatz einer organischen oder anorganischen Base, wie NEt₃, Pyridin, DMAP, Hünigbase, Na₂CO₃, CaCO₃, umsetzt.

Die Herstellung von Verbindungen der allgemeinen Formel ll, wobei A^{1'} für-CO-NR¹-(CH₂)ₙ-NR²-(CO-CHZ¹-NH)ₘ-CO-CHZ²Hal'und A^{2'} für -NR¹-CO-CHZ²Hal', mit Hal' in der Bedeutung von Chlor oder Brom, stehen erfolgt nach den dem Fachmann bekannten Verfahren der Acylierung von Aminen der allgemeinen Formel II, wobei A^{1'} für-CO-NR¹-(CH₂)ₙ-NR²-(CO-CHZ¹-NH)ₘ H und A^{2'} für -NR¹H stehen, mit Chloracetylchlorid, Chloracetylbromid, Bromacetylbromid ,2-Brompropionylbromid, 2-Brompropionylchlorid oder 2-Chlorpropionylchlorid in aprotischen Lösungsmitteln wie Acetonitril, Methylenchlorid, Chloroform, DMF, DMA, THF, Dioxan oder Toloul bei Temperaturen von 0° - 40° C gegebenenfals unter Zusatz einer organischen oder anorganischen Base, wie NEt₃, Pyridin, DMAP, Hünigbase, Na₂CO₃, CaCO₃.

Die Herstellung von Aminen der allgemeinen Formel II, wobei A^{1'} für-CO-NR¹-(CH₂)ₙ-NR²-CO-CHZ¹-NH)ₘ-H und A^{2'} für -NR¹H steht, erfolgt durch Umsetzungen von Verbindungen der allgemeinen Formel IV 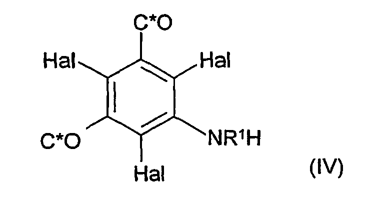 mit Diaminen der allgemeinen Formel A

HNR¹-(CH₂)ₙ-NR²H (A),

worin C*O für eine -COOH- oder aktivierte Carboxylgruppe und Hal, R¹, R², n in der oben angegebenen Bedeutung stehen, nach den dem Fachmann bekannten Methoden der Amidbildung (siehe oben) in einem aprotischen Lösungsmittel wie DMF, DMA, THF, Dioxan, 1,2-Dichlorethan, Chloroform, Dichlormethan oder Toluol gegebenenfalls unter Zusatz einer organischen oder anorganischen Base wie NEt₃, Pyridin, DMAP, Hünigbase, Na₂CO₃, K₂CO₃, CaCO₃ bei Temperaturen von 0° C -100° C.

In manchen Fällen hat es sich als vorteilhaft erwiesen, das Diamin selbst als Lösungsmittel zu verwenden. Manchmal kann es von Vorteil sein, eine der beiden endständigen Aminogruppen in geschützter Form einzusetzen (z.B. Mono-Boc, Mono-Z) und nach erfolgter Kupplung diese Schutzgruppe nach den dem Fachmann bekannten Methoden abzuspalten (T.W. Greene, s.o.).

Die Diamine bzw. monogeschützten Diamine sind literaturbekannt und käuflich zu erwerben (z.B. Aldrich, Fluka). Bevorzugt werden die Säurechloride von Verbindungen der allgemeinen Formel IV verwendet.

Die Herstellung der Verbindung 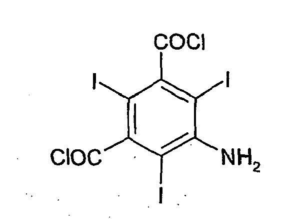 ist in DE 2943777 beschrieben.

Die Herstellung der Verbindung 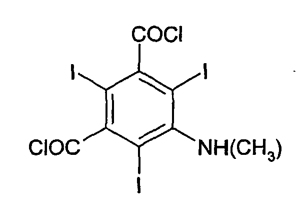 ist in EP 0033426 beschrieben.

Die Herstellung der entsprechenden Tri-Bromverbindungen erfolgt in analoger Weise wie in EP 0073715 beschrieben.

Verbindungen der allgemeinen Formel III wie z.B. Tri-Boc Cyclen, Tri-Z-Cyclen, Tri-TFA-Cyclen oder Tris-tert-butyl-DO3A sind literaturbekannt.

Die erfindungsgemäßen Verbindungen sind sowohl in der Röntgen- als auch in der MR Diagnostik einsetzbar.

Die hohe Röntgendichte gepaart mit ihrer guten Wasserlöslichkeit der halogenierten Röntgenkontrastmittel ist mit der ausgeprägten Hydrophilie der Metall-Chelate und der ihnen innewohnenden guten Verträglichkeit in einem Molekül vereint. Die sehr hohe Hydrophilie der neuen Verbindungen führt dazu, dass das Nebenwirkungsprofil dem der sehr gut verträglichen Gd-Verbindungen entspricht, wie sie in der MR-Bildgebung verwendet werden. Diese Eigenschaft macht sie daher besonders geeignet für den Einsatz bei Patienten mit einer nachgewiesenen Allergie gegen iodierte Verbindungen oder bei vorhandener Atopie. Besonders wird die Inzidenz der schweren Nebenwirkungen wie Bronchospasmus und Schock oder gar Tod auf das niedrige Niveau der MR-Kontrastmittel gesenkt.
Die geringe Osmolalität der Formulierungen ist Indiz für eine generelle sehr gute Verträglichkeit der neuen Verbindungen. Sie sind deshalb besonders für intravasale (parenterale) Anwendungen geeignet.

Je nach pharmazeutischer Formulierung können die Kontrastmittel exklusiv für die Röntgendiagnostik (Trihalogen Komplexe mit diamagnetischen Metallen) aber auch gleichzeitig für die Röntgen- und MRT Diagnostik (Trihalogen Komplexe mit paramagnetischen Atomen, vorzugsweise Gd) eingesetzt werden. Sehr vorteilhaft sind die Verbindungen z.B. in der Urographie, Computer Tomographie, Angiographie, Gastrographie, Mammographie, Kardiologie und Neuroradiologie einsetzbar. Auch bei der Strahlentherapie sind die verwendeten Komplexe von Vorteil. Die Verbindungen sind für alle Perfusionsmessungen geeignet. Eine Differenzierung von gut mit Blut versorgten und ischämischen Bereichen ist nach intravasaler Injektion möglich. Ganz allgemein können diese Verbindungen in allen Indikationen eingesetzt werden, wo konventionelle Kontrastmittel in der Röntgen- bzw. MR Diagnostik Verwendung finden.

Die neuen Kontrastmittel können ferner für die Magnetisierung-Transfer-Technik (siehe z.B. Journ. Chem.Phys. 39(11), 2892(1963), sowie WO 03/013616) Verwendung finden, soweit sie mobile Protonen in ihrer chemische Struktur enthalten.

Diagnostisch besonders wertvoll ist die Kontrastierung von Gehirninfarkten und Tumoren der Leber bzw. raumfordernden Prozessen in der Leber sowie von Tumoren des Abdomens (inklusive der Nieren) und des Muskel-Skelett-Systems. Aufgrund des niedrigen osmotischen Druckes sind besonders vorteilhaft die Blutgefäße nach intraarterieller aber auch intravenöser Injektion darstellbar.

Ist die erfindungsgemäße Verbindung zur Anwendung in der MR-Diagnostik bestimmt, so muss das Metallion der signalgebenden Gruppe paramagnetisch sein. Dies sind insbesondere die zwei- und dreiwertigen lonen der Elemente der Ordnungszahlen 21-29, 42, 44 und 58-70. Geeignete lonen sind beispielsweise das Chrom(III)-, Eisen(II)-, Kobalt(II)-, Nickel(II)-, Kupfer(II)-, Praseodym(III)-, Neodym(III)-, Samarium(III)- und Ytterbium(III)-ion. Wegen ihres starken magnetischen Moments sind bevorzugt Gadolinium(III)-, Terbium(III)-, Dysprosium(III)-, Holmium(III)-, Erbium(III)-, Eisen(III)- und Mangan(II)-ionen, besonders bevorzugt Gadolinium(III)- und Mangan(II)-ionen.

Ist die erfindungsgemäße Verbindung zur Anwendung in der Röntgen-Diagnostik bestimmt, so leitet sich das Metallion vorzugsweise von einem Element höherer Ordnungszahl ab, um eine ausreichende Absorption der Röntgenstrahlen zu erzielen. Es wurde gefunden, dass zu diesem Zweck diagnostische Mittel, die ein physiologisch verträgliches Komplexsalz mit Metallionen von Elementen der Ordnungszahlen 25, 26 und 39 sowie 57-83 enthalten, geeignet sind.

Bevorzugt sind Mangan(II)-, Eisen(II)-, Eisen(III)-, Praseodym(lll)-, Neodym(III)-, Samarium(lll)-, Gadolinium(III)-, Ytterbium(III)- oder Bismut(III)-ionen, insbesondere Dysprosium(III)-ionen und Yttrium(III)-ionen.

Die Herstellung der erfindungsgemäßen pharmazeutischen Mittel erfolgt in an sich bekannter Weise, indem man die erfindungsgemäßen Komplexverbindungen - gegebenenfalls unter Zugabe der in der Galenik üblichen Zusätze - in wässrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die zu den erfindungsgemäßen Metallkomplexen korrespondierenden Ca-Komplexe) oder - falls erforderlich - Elektrolyte wie zum Beispiel Natriumchlorid oder - falls erforderlich - Antioxidantien wie zum Beispiel Ascorbinsäure.

Sind für die enterale bzw. parenterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methyl-cellulose, Lactose, Mannit] und/oder Tensid(en) [zum Beispiel Lecithine, Tween^{®}, Myrj^{®}] und/oder Aromastoff(en) zur Geschmackskorrektur [zum Beispiel ätherischen Ölen] gemischt.

Prinzipiell ist es auch möglich, die erfindungsgemäßen pharmazeutischen Mittel ohne Isolierung der Komplexe herzustellen. In jedem Fall muss besondere Sorgfalt darauf verwendet werden, die Chelatbildung so vorzunehmen, dass die erfindungsgemäßen Komplexe praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexes.

Bei der in-vivo-Applikation der erfindungsgemäßen Mittel können diese zusammen mit einem geeigneten Träger wie zum Beispiel Serum oder physiologischer Kochsalzlösung und zusammen mit einem anderen Protein wie zum Beispiel Humanserumalbumin (HSA) verabreicht werden.

Die erfindungsgemäßen Mittel werden üblicherweise parenteral, vorzugsweise i.v., appliziert. Sie können auch intraarteriell oder interstitiell/intrakutan appliziert werden, je nachdem, ob ein Gefäß/Organ selektiv kontrastiert (z.B. Darstellung der Koronararterien nach intraarterieller Injektion) oder Gewebe bzw. Pathologien (z.B. Diagnose von Gehirntumoren nach intravenöser Injektion) dargestellt werden soll.

Die erfindungsgemäßen pharmazeutischen Mittel enthalten vorzugsweise 0,001 - 1 Mol/l der genannten Verbindung und werden in der Regel in Mengen von 0,001 - 5 mMol/kg dosiert.

Die erfindungsgemäßen Mittel erfüllen die vielfältigen Voraussetzungen für die Eignung als Kontrastmittel für die magnetische Resonanztomographie. So sind sie hervorragend dazu geeignet, nach oraler oder parenteraler Applikation durch Erhöhung der Signalintensität das mit Hilfe des MR-Tomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten. Von großem Vorteil für die Verwendung in der magnetischen Resonanztomographie ist die hohe Wirksamkeit (Relaxivity) der erfindungsgemäßen paramagnetischen Verbindungen. So ist die Relaxivity (L/mmol⁻¹*sec⁻¹ von gadoliniumhaltigen Verbindungen in der Regel zwei- bis vierfach größer als bei herkömmlichen Gd-Komplexen (z.B. Gadobutrol).

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so dass eine Freigabe oder ein Austausch der in den Komplexen gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als MRT-Diagnostika in Mengen von 0,001-5 mMol Gd/kg, vorzugsweise 0,005-0,5 mMol Gd/kg, dosiert.

Die erfindungsgemäßen Mittel sind hervorragend als Röntgenkontrastmittel geeignet, wobei besonders hervorzuheben ist, dass sich mit ihnen keine Anzeichen der von den jodhaltigen Kontrastmitteln bekannten anaphylaxieartigen Reaktionen in biochemisch-pharmakologischen Untersuchungen erkennen lassen. Im Falle der starken Röntgenabsorption sind sie besonders effektiv in Bereichen höherer Röhrenspannungen (z. B. CT und DSA).

Im allgemeinen werden die erfindungsgemäßen Mittel für die Anwendung als Röntgenkontrastmittel in Analogie zum Beispiel Meglumin-Diatrizoat in Mengen von 0,01 - 5 mMol/kg, vorzugsweise 0,02 - 1 mMol Substanz/kg, welches im Falle von z.B. lod-Dy Verbindungen 0,06-6 mMol (l+Dy)/kg entspricht, dosiert. Je nach diagnostischer Fragestellung können Formulierungen gewählt werden, die sowohl in der Röntgen- als auch in der MR Diagnostik einsetzbar sind. Um optimale Ergebnisse für beide Imaging Modalitäten zu erzielen, kann es vorteilhaft sein, Formulierungen zu wählen, in denen der Anteil paramagnetischer Ionen reduziert ist, da für viele Anwendungen der MR Diagnostik ein zu hoher Anteil paramagnetischer Ionen keinen weiteren Zugewinn liefert.
Für einen dualen Nutzen können Formulierungen eingesetzt werden, bei denen der prozentuale Anteil von paramagnetischen Stoffen (z.B. Gd) auf 0,05 bis 50, bevorzugt auf 2-20 % verringert ist. Als Beispiel sei eine Anwendung in der Herzdiagnostik erwähnt. Für die Untersuchung wird eine Formulierung bestehend aus den erfindungsgemäßen Substanzen in einer Gesamtkonzentration von z.B. 0.25 mol/L verwendet. Der Anteil Gd haltiger Komplexe ist 20 % , die restlichen 80 % der Metalle sind z.B. Dy Atome. Bei einer Röntgen Koronarangiographie nach intra-arterieller oder intravenöser Gabe werden z.B. 50 mL eingesetzt, d.h. 0.18 mMol Substanz pro kg Körpergewicht bei einem 70 kg schweren Patienten. Kurz nach erfolgter Röntgendarstellung der Herzkranzgefäße wird eine MR Diagnose des Herzens angeschlossen, um vitale von nekrotischen Myokardbezirken differenzieren zu können. Die für die Untersuchung zuvor applizierte Menge von etwa 110 µmol Gd/kg ist hierfür optimal.

### Patentbeispiele

### Beispiel 1

### a) N,N-Bis-(2-aminoethyl)-5-(methylamino)-2,4,6-triiodisophthalsäureamid

Eine Lösung von 10 g (16,4 mmol) 2,4,6-Triiod-5-(methylamino)-isophthalsäuredichlorid (EP 0033426, Sovak, 1/80 US) in 100 ml Tetrahydrofuran wird zu 26.7 ml (399 mmol) Ethylendiamin über 1 h bei Raumtemp. getropft und 14 h nachgerührt. Der ausgefallene Feststoff wird abfiltriert, mit Ethanol nachgewaschen, in 100 ml Wasser aufgenommen und mit 1 M Lithiumhydroxid-Lösung auf einen pH-Wert von 8.0 eingestellt. Nach Eindampfen im Vakuum wird aus Ethanol umkristallisiert.
Ausbeute: 8,7 g (81 % d. Th.) eines farblosen Feststoffes

| Elementaranalyse: | | | | |
|---|---|---|---|---|
| ber.: | C 23.77 | H 2.76 | N 10.66 | I 57.94 |
| gef.: | C 23.98 | H 2.71 | N 10.62 | I 57.68 |

### b) N,N-Bis-[2-(2-brompropionylamino)ethyl)-5-[(2-brompropionyl)methylamino]-2,4,6-triiodisophthalsäureamid

50 g (76,1 mmol) *N,N-*Bis-(2-aminoethyl)-5-(methylamino)-2,4,6-triiodisophthalsäureamid werden in 500 ml Dimethylacetamid gelöst und bei 0 °C 75,5 g (350 mmol) 2-Brompropionsäurebromid (Aldrich) über 15 min zugetropft. Anschließend wird 20 h bei 40 °C gerührt. Die Reaktionsmischung wird in 4000 ml Eiswasser gegossen, der anfallende Feststoff abfiltriert, in 800 ml Ethylacetat gelöst und dreimal mit je 250 ml Wasser extrahiert. Die organische Phase wird über Natriumsulfat getrocknet, das Lösungsmittel zur Trockene eingeengt. Das Rohprodukt wird aus Methyl*-tert*-butylether umkristallisiert.
Ausbeute: 57 g (71 % d. Th.) eines farblosen Feststoffes

| Elementaranalyse: | | | |
|---|---|---|---|
| ber.: | C 24.88 | H 2.56 | N 6.60 |
| gef.: | C 25.21 | H 2.63 | N 6.71 |

### c) 2,4,6-Triiod-5-(2-{10-[1,4,7-tris-(benzyloxycarbonyl)-1,4,7,10-tetraazacyclododecanyl]}-propionyl)methylaminoisophthalsäure-N,N-bis-(3-aza-5-methyl-4-oxopentan-1,5-diyl-{10-[1,4,7-tris-(benzyloxycarbonyl)-1,4,7,10-tetraazacyclododecanyl]})amid

111.6 g (194.2 mmol) 1,4,7-Tris-(benzyloxycarbonyl)-1,4,7,10-tetrazacyclododecan (Delaney et al. , J. Chem. Soc. Perkin Trans. 1991, 3329) werden in 800 ml Acetonitril gelöst und mit 53,7 g (388,4 mmol) Natriumcarbonat versetzt. Anschließend werden unter starkem Rühren 55 g (51.8 mmol) *N*,*N*-Bis-[2-(2-brompropionylamino)ethyl)-5-[(2-brompropionyl)methylamino]-2,4,6-triiodisophthalsäureamid zugegeben und 20 h unter Rückfluß erhitzt. Es wird von unlöslichen Bestandteilen abfiltriert, zur Trockene eingeengt und der Rückstand an Kieselgel chromatographiert (Laufmittel Dichlormethan/Methanol 20:1). Die das Produkt enthaltenden Fraktionen werden vereinigt und eingedampft.
Ausbeute 51,1 g (39 % d. Th.) eines farblosen Feststoffes

| Elementaranalyse: | | | | |
|---|---|---|---|---|
| ber.: | C 55.73 | H 5.47 | N 9.36 | I 14.97 |
| gef.: | C 55.91 | H 5.44 | N 9.38 | I 14.89 |

### d) 2,4,6-Triiod-5-[2-(1,4,7,10-tetraazacyclododecanyl)propionyl]methylaminoisophthalsäure-N,N-bis-[3-aza-5-methyl-4-oxopentan-1,5-diyl-(1,4,7,10-tetraazacyclododecan-yl)]amid

50 g (19,7 mmol) 2,4,6-Triiod-5-(2-{10-[1,4,7-tris-(benzyloxycarbonyl)-1,4, 7,10-tetraazacyclododecanyl]}propionyl)methylaminoisophthalsäure-*N*,*N*- bis-(3-aza-5-methyl-4-oxopentan-1,5-diyl-{10-[1,4,7-tris-(benzyloxycarbonyl)-1,4,7,10-tetraazacyclodo-decanyl]})amid werden bei 0-5°C vorsichtig mit 500 ml HBr/AcOH (33%) versetzt und 3 h bei Raumtemp. gerührt. Anschließend wird die Reaktionsmischung in 2500 ml Diethylether gegossen, der dabei anfallende Feststoff abgesaugt und mit Diethylether mehrfach nachgewaschen. Der Rückstand wird in 300 ml Wasser und 300 ml Dichlormethan gelöst unter starkem Rühren gibt man so lange 32proz. NaOH-Lösung bis ein pH-Wert von 10 erreicht ist. Die organische Phase wird abgetrennt, die wässrige Phase dreimal mit je 150 ml Dichlormethan extrahiert, die vereinigten organischen Phasen über Magnesiumsulfat getrocknet und zur Trockene eingeengt.
Ausbeute 24,4 g (93 % d. Th.) eines farblosen Feststoffes

| Elementaranalyse: | | | | |
|---|---|---|---|---|
| ber.: | C 41.36 | H 6.34 | N 17.82 | I 28.50 |
| gef.: | C 41.49 | H 6.37 | N 17.76 | I 28.39 |

### e) 2,4,6-Triiod-5-(2-{10-[1,4,7-tris-(carboxymethyl)-1,4,7,10-tetraazacyclododecanyl]}propionyl)methylaminoisophthalsäure-N,N-bis-(3-aza-5-methyl-4-oxopentan-1,5-diyl-{10-[1,4,7-tris-(carboxymethyl)-1,4,7,10-tetraazacyclododecanyl]})amid

23,8 g (17,8 mmol) 2,4,6-Triiod-5-[2-(1,4,7,10-tetraazacyclododecanyl)propionyl]-methylaminoisophthalsäure-N,N-bis-[3-aza-5-methyl-4-oxopentan-1,5-diyl-(1,4,7,10-tetraazacyclododecan-yl)]amid werden in 200 ml Wasser gelöst, 25,7 g (272,7 mmol) Chloressigsäure hinzugegeben und bei 60 °C mit 32 %iger NaOH ein pH-Wert von 9.5 eingestellt. Es wird 10 h auf 70 °C erhitzt, wobei man den pH-Wert der Reaktionsmischung kontinuierlich auf 9.5 nachstellt. Nach Abkühlen auf Raumtemp. wird mit konz. HCl ein pH-Wert von 1 eingestellt und die Lösung im Vakuum eingedampft. Der Rückstand wird mit 300 ml Methanol ausgerührt, von unlöslichen Bestandteilen abfiltriert und das Filtrat eingedampft. Der Rückstand wird in 200 ml Wasser gelöst und auf eine lonenaustauscher-Säule (1200 ml, IR 120, H⁺-form) gegeben. Anschließend wird mit 5 I Wasser gewaschen und das saure Eluat eingedampft. Der Rückstand wird in 150 ml Methanol gelöst und in 2500 ml Diethylether getropft, der dabei anfallende Feststoff abgesaugt, mit Diethylether mehrfach nachgewaschen und im Vakuum getrocknet.
Ausbeute 24,1 g (73 % d. Th.) eines farblosen Feststoffes

| Elementaranalyse: | | | | |
|---|---|---|---|---|
| ber.: | C 41.37 | H 5.53 | N 12.81 | I 20.49 |
| gef.: | C 41.54 | H 5.57 | N 12.77 | I 20.31 |

### f) 2,4,6-Triiod-5-(2-{10-[1,4,7-tris-(carboxymethyl)-1,4,7,10-tetraazacyclododecanyl]}propionyl, Gd-Komplex)methylaminoisophthalsäure-N,N-bis-(3-aza-5-methyl-4-oxopentan-1,5-diyl-{10-[1,4,7-tris-(carboxymethyl)-1,4,7,10-tetraazacyclododecanyl, Gd-Komplex]})amid

12,8 g (6,9 mmol) 2,4,6-Triiod-5-(2-{10-[1,4,7-tris-(carboxymethyl)-1,4,7,10-tetraazacyclododecanyl]}propionyl)methylaminoisophthalsäure-*N,N*-bis-(3-aza-5-methyl-4-oxopentan-1,5-diyl-{10-[1,4,7-tris-(carboxymethyl)-1,4,7,10-tetraazacyclododecanyl]})amid werden in 100 ml Wasser gelöst und durch Zugabe von 3 ml Essigsäure angesäuert. Es werden 3,7 g (10,4 mmol) Gadoliniumoxid zugegeben und 6 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 10/10/1). Die das Produkt enthaltenden Fraktionen werden vereinigt und mit 10 g Ionenaustauscher (IR 267 H-Form) 2 h ausgerührt und abfiltriert, dann mit 10 g Ionenaustauscher (IRA 67 OH-Form) 2 h ausgerührt, abfiltriert, mit 2 g Aktivkohle versetzt, 2 h auf 60 °C erwärmt, abfiltriert und gefriergetrocknet.
Ausbeute 9,9 g (58 % d. Th.) eines farblosen Feststoffes
Wassergehalt (Karl-Fischer): 6,4 %

| Elementaranalyse (bezogen auf die wasserfreie Substanz): | | | | | |
|---|---|---|---|---|---|
| ber.: | C 33.12 | H 4.04 | N 10.26 | l 16.40 | Gd 20.33 |
| gef.: | C 33.31 | H 4.08 | N 10.23 | l 16.31 | Gd 20.27 |

### Beispiel 2

### 2,4,6-Triiod-5-(2-{10-[1,4,7-tris-(carboxymethyl)-1,4,7,10-tetraazacyclododecanyl]}propionyl, Dy-Komplex)methylaminoisophthalsäure-N,N-bis-(3-aza-5-methyl-4-oxopentan-1,5-diyl-{10-[1,4,7-tris-(carboxymethyl)-1,4,7,10-tetraazacyclododecanyl, Dy-Komplex]})amid

12,8 g (6,9 mmol) 2,4,6-Triiod-5-(2-(10-[1,4,7-tris-(carboxymethyl)-1,4,7,10-tetraazacyclododecanyl]}propionyl)methylaminoisophthalsäure-*N*,*N*-bis-(3-aza-5-methyl-4-oxopentan-1,5-diyl-{10-[1,4,7-tris-(carboxymethyl)-1,4,7,10-tetraazacyclododecanyl]})amid (Titelverbindung 1e) werden in 100 ml Wasser gelöst und durch Zugabe von 3 ml Essigsäure angesäuert. Es werden 3,9 g (10,4 mmol) Dysprosiumoxid zugegeben und 6 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 10/10/1). Die das Produkt enthaltenden Fraktionen werden vereinigt und mit 10 g Ionenaustauscher (IR 267 H-Form) 2 h ausgerührt und abfiltriert, dann mit 10 g Ionenaustauscher (IRA 67 OH-Form) 2 h ausgerührt, abfiltriert, mit 2 g Aktivkohle versetzt, 2 h auf 60 °C erwärmt, abfiltriert und gefriergetrocknet.
Ausbeute 11,2 g (64 % d. Th.) eines farblosen Feststoffes
Wassergehalt (Karl-Fischer): 7,5 %

| Elementaranalyse (bezogen auf die wasserfreie Substanz): | | | | | |
|---|---|---|---|---|---|
| ber.: | C 32.90 | H 4.01 | N 10.19 | I 16.29 | Dy 20.86 |
| gef.: | C 33.03 | H 4.05 | N 10.17 | I 16.22 | Dy 20.65 |

### Beispiel 3

### 2,4,6-Triiod-5-(2-{10-[1,4,7-tris-(carboxymethyl)-1,4,7,10-tetraazacyclododecanyl]}propionyl, Yb-Komplex)methylaminoisophthalsäure-N,N-bis-(3-aza-5-methyl-4-oxopentan-1,5-diyl-{10-[1,4,7-tris-(carboxymethyl)-1,4,7,10-tetraazacyclododecanyl, Yb-Kompiex]})amid

12,8 g (6,9 mmol) 2,4,6-Triiod-5-(2-{10-[1,4,7-tris-(carboxymethyl)-1,4,7,10-tetraazacyclododecanyl]}propionyl)methylaminoisophthafsäure-*N*,*N* bis-(3-aza-5-methyl-4-oxopentan-1,5-diyl-{10-[1,4,7-tris-(carboxymethyl)-1,4,7,10-tetraazacyclododecanyl]})amid (Titelverbindung 1e) werden in 100 ml Wasser gelöst und durch Zugabe von 3 ml Essigsäure angesäuert. Es werden 4,1 g (10,4 mmol) Ytterbiumoxid zugegeben und 6 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 10/10/1).

Die das Produkt enthaltenden Fraktionen werden vereinigt und mit 10 g lonenaustauscher (IR 267 H-Form) 2 h ausgerührt und abfiltriert, dann mit 10 g Ionenaustauscher (IRA 67 OH-Form) 2 h ausgerührt, abfiltriert, mit 2 g Aktivkohle versetzt, 2 h auf 60 °C erwärmt, abfiltriert und gefriergetrocknet.
Ausbeute 9,5 g (54 % d. Th.) eines farblosen Feststoffes
Wassergehalt (Karl-Fischer): 6,7 %

| Elementaranalyse (bezogen auf die wasserfreie Substanz): | | | | | |
|---|---|---|---|---|---|
| ber.: | C 32.46 | H 3.96 | N 10.05 | I 16.07 | Yb 21.92 |
| gef.: | C 32.61 | H 4.00 | N 9.97 | I 15.98 | Yb 21.79 |

### Beispiel 4

### 2,4,6-Triiod-5-(2-{10-[1,4,7-tris-(carboxymethyl)-1,4,7,10-tetraazacydododecanyl]}propionyl, Y-Komplex)methylaminoisophthalsäure-N,N-bis-(3-aza-5-methyl-4-oxopentan-1,5-diyl-{10-[1,4,7-tris-(carboxymethyl)-1,4,7,10-tetraazacyclododecanyl, Y-Komplex]})amid

12,8 g (6,9 mmol) 2,4,6-Triiod-5-(2-{10-[1,4,7-tris-(carboxymethyl)-1,4,7,10-tetraazacyclododecanyl]}propionyl)methylaminoisophthalsäure-*N*,*N*- bis-(3-aza-5-methyl-4-oxopentan-1,5-diyl-{10-[1,4,7-tris-(carboxymethyl)-1,4,7,10-tetraazacyclododecanyl]})amid (Titelverbindung 1e) werden in 100 ml Wasser gelöst und durch Zugabe von 3 ml Essigsäure angesäuert. Es werden 2,35 g (10,4 mmol) Yttriumoxid zugegeben und 6 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 10/10/1). Die das Produkt enthaltenden Fraktionen werden vereinigt und mit 10 g lonenaustauscher (IR 267 H-Form) 2 h ausgerührt und abfiltriert, dann mit 10 g lonenaustauscher (IRA 67 OH-Form) 2 h ausgerührt, abfiltriert, mit 2 g Aktivkohle versetzt, 2 h auf 60 °C erwärmt, abfiltriert und gefriergetrocknet.
Ausbeute 7,8 g (50 % d. Th.) eines farblosen Feststoffes
Wassergehalt (Karl-Fischer) : 5,9 %

| Elementaranalyse (bezogen auf die wasserfreie Substanz): | | | | | |
|---|---|---|---|---|---|
| ber.: | C 36.33 | H 4.43 | N 11.25 | I 17.99 | Y 12.60 |
| gef.: | C 36.52 | H 4.49 | N 11.28 | I 17.81 | Y 12.40 |

### Beispiel 5

### a) N,N-Bis-[2-(bromacetylamino)ethyl)-5-[(bromacetyl)methylamino]-2,4,6-triiodisophthalsäureamid

50 g (76,1 mmol) *N*,*N*- Bis-(2-aminoethyl)-5-(methylamino)-2,4,6-triiodisophthalsäureamid (Titelverbindung 1a) werden in 500 ml Dimethylacetamid gelöst und bei 0 °C 70,6 g (350 mmol) Bromacetylbromid (Aldrich) über 15 min zugetropft. Anschließend wird 20 h bei 40 °C gerührt. Die Reaktionsmischung wird in 4000 ml Eiswasser gegossen, der anfallende Feststoff abfiltriert, in 800 ml Ethylacetat gelöst und dreimal mit je 250 ml Wasser extrahiert. Die organische Phase wird über Natriumsulfat getrocknet, das Lösungsmittel zur Trockene eingeengt. Das Rohprodukt wird aus Methyl-*tert*-butylether umkristallisiert.
Ausbeute: 60 g (77 % d. Th.) eines farblosen Feststoffes

| Elementaranalyse: | | | |
|---|---|---|---|
| ber.: | C 22.38 | H 2.08 | N 6.87 |
| gef.: | C 22.63 | H 2.21 | N 6.75 |

### b) 2,4,6-Triiod-5-({10-[1,4,7-tris-(benzyloxycarbonyl)-1,4,7,10-tetraazacyclododecanyl]}-acetyl)methylaminoisophthalsäure-N,N-bis-(3-aza-4-oxopentan-1,5-diyl-{10-[1,4,7-tris-(benzyloxycarbonyl)-1,4,7,10-tetraazacyclododecanyl]})amid

122,5 g (213,2 mmol) 1,4,7-Tris-(benzyloxycarbonyl)-1,4,7,10-tetrazacyclododecan (Delaney et al., J. Chem. Soc. Perkin Trans. 1991, 3329) werden in 800 ml Acetonitril gelöst und mit 60 g (426,4 mmol) Natriumcarbonat versetzt. Anschließend werden unter starkem Rühren 58 g (56,9 mmol) *N,N*-Bis-[2-(bromacetylamino)ethyl)-5-[(bromacetyl)methylamino]-2,4,6-triiodisophthalsäureamid zugegeben und 20 h unter Rückfluß erhitzt. Es wird von unlöslichen Bestandteilen abfiltriert, zur Trockene eingeengt und der Rückstand an Kieselgel chromatographiert (Laufmittel Dichlormethan/Methanol 20:1). Die das Produkt enthaltenden Fraktionen werden vereinigt und eingedampft.
Ausbeute 142 g (54 % d. Th.) eines farblosen Feststoffes

| Elementaranalyse: | | | | |
|---|---|---|---|---|
| ber.: | C 55.23 | H 5.32 | N 9.52 | l 15.22 |
| gef.: | C 55.41 | H 5.35 | N 9.48 | l 15.13 |

### c) 2,4,6-Triiod-5-[(1,4,7,10-tetraazacyclododecanyl)acetyl]methylaminoisophthalsäure-N,N-bis-[3-aza-4-oxopentan-1,5-diyl-(1,4,7,10-tetraazacyclododecanyl)]amid

100 g (40 mmol) 2,4,6-Triiod-5-({10-[1,4,7-tris-(benzyloxycarbonyl)-1,4,7,10-tetraazacyclododecanyl]}acetyl)methylaminoisophthalsäure-*N,N*-bis-(3-aza-4-oxopentan-1,5-diyl-{10-[1,4,7-tris-(benzyloxycarbonyl)-1,4,7,10-tetraazacyclododecanyl]})amid werden bei 0-5°C vorsichtig mit 800 ml HBr/AcOH (33%) versetzt und 3 h bei Raumtemp. gerührt. Anschließend wird die Reaktionsmischung in 4000 ml Diethylether gegossen, der dabei anfallende Feststoff abgesaugt und mit Diethylether mehrfach nachgewaschen. Der Rückstand wird in 500 ml Wasser und 500 ml Dichlormethan gelöst unter starkem Rühren gibt man so lange 32proz. NaOH-Lösung bis ein pH-Wert von 10 erreicht ist. Die organische Phase wird abgetrennt, die wässrige Phase dreimal mit je 250 ml Dichlormethan extrahiert, die vereinigten organischen Phasen über Magnesiumsulfat getrocknet und zur Trockene eingeengt.
Ausbeute 48,7 g (94 % d. Th.) eines farblosen Feststoffes

| Elementaranalyse: | | | |
|---|---|---|---|
| ber.: C 39.92 | H 6.08 | N 18.40 | I 29.42 |
| gef.: C 40.19 | H 6.13 | N 18.36 | I 29.27 |

### d) 2,4,6-Triiod-5-({10-[1,4,7-tris-(carboxymethyl)-1,4,7,10-tetraazacyclododecanyl]}acetyl)methylaminoisophthalsäure-N,N-bis-(3-aza-4-oxopentan-1,5-diyl-{10-[1,4,7-tris-(carboxymethyl)-1,4,7,10-tetraazacyclododecanyl]})amid

45 g (34,8 mmol) 2,4,6-Triiod-5-[(1,4,7,10-tetraazacyclododecanyl)acetyl]methylaminoisophthalsäure-*N,N*-bis-[3-aza-4-oxopentan-1,5-diyl-(1,4,7,10-tetraazacyclododecanyl)]amid werden in 400 ml Wasser gelöst, 50,2 g (532,8 mmol) Chloressigsäure hinzugegeben und bei 60 °C mit 32 %iger NaOH ein pH-Wert von 9.5 eingestellt. Es wird 10 h auf 70 °C erhitzt, wobei man den pH-Wert der Reaktionsmischung kontinuierlich auf 9.5 nachstellt. Nach Abkühlen auf Raumtemp. wird mit konz. HCl ein pH-Wert von 1 eingestellt und die Lösung im Vakuum eingedampft. Der Rückstand wird mit 300 ml Methanol ausgerührt, von unlöslichen Bestandteilen abfiltriert und das Filtrat eingedampft. Der Rückstand wird in 300 ml Wasser gelöst und auf eine lonenaustauscher-Säule (1200 ml, IR 120, H⁺-form) gegeben. Anschließend wird mit 5 I Wasser gewaschen und das saure Eluat eingedampft. Der Rückstand wird in 250 ml Methanol gelöst und in 4500 ml Diethylether getropft, der dabei anfallende Feststoff abgesaugt, mit Diethylether mehrfach nachgewaschen und im Vakuum getrocknet.
Ausbeute 49,9 g (79 % d. Th.) eines farblosen Feststoffes

| Elementaranalyse: | | | | |
|---|---|---|---|---|
| ber.: | C 40.34 | H 5.33 | N 13.11 | I 20.96 |
| gef.: C | C 40.57 | H 5.34 | N 13.07 | I 20.77 |

### e) 2,4,6-Triiod-5-({10-[1,4,7-tris-(carboxymethyl)-1,4,7,10-tetraazacyclododecanyl]}acetyl, Gd-Komplex)methylaminoisophthalsäure-N,N-bis-(3-aza-4-oxopentan-1,5-diyl-{10-[1,4,7-tris-(carboxymethyl)-1,4,7,10-tetraazacyclododecanyl, Gd-Komplex]})amid

12,5 g (6,9 mmol) 2,4,6-Triiod-5-({10-[1,4,7-tris-(carboxymethyl)-1,4,7,10-tetraazacydododecanyl]}acetyl)methylaminoisophthalsäure-*N*,*N*-bis-(3-aza-4-oxopentan-1,5-diyl-{10-[1,4,7-tris-(carboxymethyl)-1,4,7,10-tetraazacyclododecanyl]})amid werden in 100 ml Wasser gelöst und durch Zugabe von 3 ml Essigsäure angesäuert. Es werden 3,7 g (10,4 mmol) Gadoliniumoxid zugegeben und 6 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 10/10/1). Die das Produkt enthaltenden Fraktionen werden vereinigt und mit 10 g lonenaustauscher (IR 267 H-Form) 2 h ausgerührt und abfiltriert, dann mit 10 g lonenaustauscher (IRA 67 OH-Form) 2 h ausgerührt, abfiltriert, mit 2 g Aktivkohle versetzt, 2 h auf 60 °C erwärmt, abfiltriert und gefriergetrocknet.
Ausbeute 8,7 g (52 % d. Th.) eines farblosen Feststoffes
Wassergehalt (Karl-Fischer): 5,8 %

| Elementaranalyse (bezogen auf die wasserfreie Substanz): | | | | | |
|---|---|---|---|---|---|
| ber.: | C 32.15 | H 3.85 | N 10.45 | I 16.71 | Gd 20.70 |
| gef.: | C 32.39 | H 3.88 | N 10.51 | I 16.59 | Gd 20.51 |

### Beispiel 6

### a) N,N-Bis-(2-aminoethyl)-5-amino-2,4,6-triiodisophthalsäureamid

Eine Lösung von 10 g (16,8 mmol) 5-Amino-2,4,6-triodisophthalsäuredichlorid (DE 2943777, Schering AG, (Priorität: 26.10.79)) in 100 ml Tetrahydrofuran wird zu 26.7 ml (399 mmol) Ethylendiamin über 1 h bei Raumtemp. getropft und 14 h nachgerührt. Der ausgefallene Feststoff wird abfiltriert, mit Ethanol nachgewaschen, in 100 ml Wasser aufgenommen und mit 1 M Lithiumhydroxid-Lösung auf einen pH-Wert von 8.0 eingestellt. Nach Eindampfen im Vakuum wird aus Ethanol umkristallisiert.
Ausbeute: 8,3 g (77 % d. Th.) eines farblosen Feststoffes

| Elementaranalyse: | | | | |
|---|---|---|---|---|
| ber.: | C 22.42 | H 2.51 | N 10.89 | I 59.21 |
| gef.: | C 22.84 | H 2.62 | N 10.99 | I 58.89 |

### b) N,N-Bis-[2-(2-brompropionylamino)ethyl)-5-[(2-brompropionyl)amino]-2,4,6-triiodisophthalsäureamid

50 g (77,8 mmol) *N,N-*Bis-(2-aminoethyl)-5-amino-2,4,6-triiodisophthalsäureamid werden in 500 ml Dimethylacetamid gelöst und bei 0°C 75,5 g (350 mmol) 2-Brompropionsäurebromid (Aldrich) über 15 min zugetropft. Anschließend wird 20 h bei 40 °C gerührt. Die Reaktionsmischung wird in 4000 ml Eiswasser gegossen, der anfallende Feststoff abfiltriert, in 800 ml Ethylacetat gelöst und dreimal mit je 250 ml Wasser extrahiert. Die organische Phase wird über Natriumsulfat getrocknet, das Lösungsmittel zur Trockene eingeengt. Das Rohprodukt wird aus Methyl-*tert*-butylether umkristallisiert.
Ausbeute: 45 g (55 % d. Th.) eines farblosen Feststoffes

| Elementaranalyse: | | | |
|---|---|---|---|
| ber.: | C 24.07 | H 2.40 | N 6.68 |
| gef.: | C 24.29 | H 2.46 | N 6.58 |

### c) 2,4,6-Triiod-5-(2-{10-[1,4,7-tris-(benzyloxycarbonyl)-1,4,7,10-tetraazacyclododecanyl]}-propionyl)aminoisophthalsäure-N,N-bis-(3-aza-5-methyl-4-oxopentan-1,5-diyl-{10-[1,4,7 -tris-(benzyloxycarbonyl)-1,4,7,10-tetraazacyclododecanyl]})amid

90,5 g (157,5 mmol) 1,4,7-Tris-(benzyloxycarbonyl)-1,4,7,10-tetrazacyclododecan (Delaney et al. , J. Chem. Soc. Perkin Trans. 1991, 3329) werden in 800 ml Acetonitril gelöst und mit 43,6 g (315 mmol) Natriumcarbonat versetzt. Anschließend werden unter starkem Rühren 44 g (42 mmol) *N*,*N* Bis-[2-(2-brompropionylamino)ethyl)-5-[(2-brompropionyl)amino]-2,4,6-triiodisophthalsäureamid zugegeben und 20 h unter Rückfluß erhitzt. Es wird von unlöslichen Bestandteilen abfiltriert, zur Trockene eingeengt und der Rückstand an Kieselgel chromatographiert (Laufmittel Dichlormethan/Methanol 20 : 1). Die das Produkt enthaltenden Fraktionen werden vereinigt und eingedampft.
Ausbeute 48,9 g (46 % d. Th.) eines farblosen Feststoffes

| Elementaranalyse: | | | | |
|---|---|---|---|---|
| ber.: | C 55.56 | H 5.42 | N 9.41 | I 14.05 |
| gef.: | C 55.72 | H 5.44 | N 9.32 | I 13.96 |

### d) 2,4,6-Triiod-5-[2-(1,4,7,10-tetraazacyclododecanyl)propionyl]aminoisophthalsäure-N,N-bis-[3-aza-5-methyl-4-oxopentan-1,5-diyl-(1,4,7,10-tetraazacyclododecanyl)]amid

48 g (19 mmol) 2,4,6-Triiod-5-(2-{10-[1,4,7-tris-(benzyloxycarbonyl)-1,4,7,10-tetraazacyclododecanyl]}propionyl)aminoisophthalsäure-*N,N-*bis-(3-aza-5-methyl-4-oxopentan-1,5-diyl-{10-[1,4,7-tris-(benzyloxycarbonyl)-1,4,7,10-tetraazacyclododecanyl]})amid werden bei 0-5°C vorsichtig mit 500 ml HBr/AcOH (33%) versetzt und 3 h bei Raumtemp. gerührt. Anschließend wird die Reaktionsmischung in 2500 ml Diethylether gegossen, der dabei anfallende Feststoff abgesaugt und mit Diethylether mehrfach nachgewaschen. Der Rückstand wird in 300 ml Wasser und 300 ml Dichlormethan gelöst unter starkem Rühren gibt man so lange 32proz. NaOH-Lösung bis ein pH-Wert von 10 erreicht ist. Die organische Phase wird abgetrennt, die wässrige Phase dreimal mit je 150 ml Dichlormethan extrahiert, die vereinigten organischen Phasen über Magnesiumsulfat getrocknet und zur Trockene eingeengt.
Ausbeute 24,3 g (97 % d. Th.) eines farblosen Feststoffes

| Elementaranalyse: | | | | |
|---|---|---|---|---|
| ber.: | C 40.89 | H 6.25 | N 18.01 | I 28.80 |
| gef.: | C 41.04 | H 6.27 | N 17.96 | I 28.63 |

### e) 2,4,6-Triiod-5-(2-{10-[1,4,7-tris-(carboxymethyl)-1,4,7,10-tetraazacyclododecanyl]}-propionyl)aminoisophthalsäure-N,N-bis-(3-aza-5-methyl-4-oxopentan-1,5-diyl-{10-[1,4,7-tris-(carboxymethyl)-1,4,7,10-tetraazacyclododecanyl]})amid

20 g (15.1 mmol) 2,4,6-Triiod-5-[2-(1,4,7,10-tetraazacyclododecanyl)propionyl]aminoisophthalsäure-*N,N*-bis-[3-aza-5-methyl-4-oxopentan-1,5-diyl-(1,4,7,10-tetraazacyclododecanyl)]amid werden in 200 ml Wasser gelöst, 21,8 g (231,8 mmol) Chloressigsäure hinzugegeben und bei 60 °C mit 32 %iger NaOH ein pH-Wert von 9.5 eingestellt. Es wird 10 h auf 70 °C erhitzt, wobei man den pH-Wert der Reaktionsmischung kontinuierlich auf 9.5 nachstellt. Nach Abkühlen auf Raumtemp. wird mit konz. HCl ein pH-Wert von 1 eingestellt und die Lösung im Vakuum eingedampft. Der Rückstand wird mit 300 ml Methanol ausgerührt, von unlöslichen Bestandteilen abfiltriert und das Filtrat eingedampft. Der Rückstand wird in 200 ml Wasser gelöst und auf eine lonenaustauscher-Säule (1200 ml, IR 120, H⁺-form) gegeben. Anschließend wird mit 5 l Wasser gewaschen und das saure Eluat eingedampft. Der Rückstand wird in 150 ml Methanol gelöst und in 2500 ml Diethylether getropft, der dabei anfallende Feststoff abgesaugt, mit Diethylether mehrfach nachgewaschen und im Vakuum getrocknet.
Ausbeute 18,7 g (67 % d. Th.) eines farblosen Feststoffes

| Elementaranalyse: | | | | |
|---|---|---|---|---|
| ber.: | C 41.03 | H 5.47 | N 12.91 | I 20.64 |
| gef.: | C 41.27 | H 5.52 | N 12.88 | 120.55 |

### f) 2,4,6-Triiod-5-(2-{10-[1,4,7-tris-(carboxymethyl)-1,4,7,10-tetraazacyclododecanyl]}propionyl, Gd-Komplex)aminoisophthalsäure-N,N-bis-(3-aza-5-methyl-4-oxopentan-1,5-diyl-{10-[1,4,7-tris-(carboxymethyl)-1,4,7,10-tetraazacyclododecanyl, Gd-Komplex]})amid

12,7 g (6,9 mmol) 2,4,6-Triiod-5-(2-{10-[1,4,7-tris-(carboxymethyl)-1,4,7,10-tetraazacyclododecanyl]}propionyl)aminoisophthalsäure-*N,N*-bis-(3-aza-5-methyl-4-oxopentan-1,5-diyl-{10-[1,4,7-tris-(carboxymethyl)-1,4,7,10-tetraazacyclododecanyl]})amid werden in 100 ml Wasser gelöst und durch Zugabe von 3 ml Essigsäure angesäuert. Es werden 3,7 g (10,4 mmol) Gadoliniumoxid zugegeben und 6 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 10/10/1). Die das Produkt enthaltenden Fraktionen werden vereinigt und mit 10 g lonenaustauscher (IR 267 H-Form) 2 h ausgerührt und abfiltriert, dann mit 10 g lonenaustauscher (IRA 67 OH-Form) 2 h ausgerührt, abfiltriert, mit 2 g Aktivkohle versetzt, 2 h auf 60 °C erwärmt, abfiltriert und gefriergetrocknet.
Ausbeute 11 g (64 % d. Th.) eines farblosen Feststoffes
Wassergehalt (Karl-Fischer): 7,3 %

| Elementaranalyse (bezogen auf die wasserfreie Substanz): | | | | | |
|---|---|---|---|---|---|
| ber.: | C 32.80 | H 3.98 | N 10.32 | l 16.50 | Gd 20.45 |
| gef.: | C 33.02 | H 4.00 | N 10.28 | l 16.43 | Gd 20.31 |

### Beispiel 7

### a) N,N-Bis-[2-(bromacetylamino)ethyl)-5-[(bromacetyl)amino]-2,4,6-triiodisophthalsäureamid

50 g (77,8 mmol) *N,N*-Bis-(2-aminoethyl)-5-amino-2,4,6-triiodisophthalsäureamid (Titelverbindung 6a) werden in 500 ml Dimethylacetamid gelöst und bei 0 °C 70,6 g (350 mmol) Bromacetylbromid (Aldrich) über 15 min zugetropft. Anschließend wird 20 h bei 40 °C gerührt. Die Reaktionsmischung wird in 4000 ml Eiswasser gegossen, der anfallende Feststoff abfiltriert, in 800 ml Ethylacetat gelöst und dreimal mit je 250 ml Wasser extrahiert. Die organische Phase wird über Natriumsulfat getrocknet, das Lösungsmittel zur Trockene eingeengt. Das Rohprodukt wird aus Methyl-*tert*-butylether umkristallisiert.
Ausbeute: 60 g (77 % d. Th.) eines farblosen Feststoffes
Ausbeute: 33,1 g (42 % d. Th.) eines farblosen Feststoffes

| Elementaranalyse: | | | |
|---|---|---|---|
| ber.: | C 21.50 | H 1.90 | N 6.96 |
| gef.: | C 27.72 | H 1.97 | N 6.86 |

### b) 2,4,6-Triiod-5-({10-[1,4,7-tris-(benzyloxycarbonyl)-1,4,7,10-tetraazacyclododecanyl]}-acetyl)aminoisophthalsäure-N,N bis-(3-aza-4-oxopentan-1,5-diyl-{10-[1,4,7-tris-(benzyloxycarbonyl)-1,4,7,10-tetraazacyclododecanyl]})amid

68,5 g (199,2 mmol) 1,4,7-Tris-(benzyloxycarbonyl)-1,4,7,10-tetrazacyclododecan (Delaney et al. , J. Chem. Soc. Perkin Trans. 1991, 3329) werden in 500 mi Acetonitril gelöst und mit 33,5 g (238,4 mmol) Natriumcarbonat versetzt. Anschließend werden unter starkem Rühren 32 g (31,8 mmol) *N,N*-Bis-[2-(bromacetylamino)ethyl)-5-[(bromacetyl)amino]-2,4,6-trüodisophthalsäureamid zugegeben und 20 h unter Rückfluß erhitzt. Es wird von unlöslichen Bestandteilen abfiltriert, zur Trockene eingeengt und der Rückstand an Kieselgel chromatographiert (Laufmittel Dichlormethan/Methanol 20 : 1). Die das Produkt enthaltenden Fraktionen werden vereinigt und eingedampft.
Ausbeute 60.7 g (77 % d. Th.) eines farblosen Feststoffes

| Elementaranalyse: | | | | |
|---|---|---|---|---|
| ber.: | C 55.05 | H 5.27 | N 9.57 | I 15.31 |
| gef.: | C 55.22 | H 5.24 | N 9.51 | I 15.23 |

### c) 2,4,6-Triiod-5-[(1,4,7,10-tetraazacyclododecanyl)acetyl]aminoisophthalsäure-N,N-bis-[3-aza-4-oxopentan-1,5-diyl-(1,4,7,10-tetraazacyclododecanyl)]amid

60 g (24,1 1 mmol) 2,4,6-Triiod-5-({10-[1,4,7-tris-(benzyloxycarbonyl)-1,4,7,10-tetraazacyciododecanyl]}acetyl)aminoisophthalsäure-*N,N* bis-(3-aza-4-oxopentan-1,5-diyl-{10-[1,4,7-tris-(benzyloxycarbonyl)-1,4,7,10-tetraazacyclododecanyl]})amid werden bei 0-5°C vorsichtig mit 500 ml HBr/AcOH (33%) versetzt und 3 h bei Raumtemp. gerührt. Anschließend wird die Reaktionsmischung in 2500 ml Diethylether gegossen, der dabei anfallende Feststoff abgesaugt und mit Diethylether mehrfach nachgewaschen. Der Rückstand wird in 300 ml Wasser und 300 ml Dichlormethan gelöst unter starkem Rühren gibt man so lange 32proz. NaOH-Lösung bis ein pH-Wert von 10 erreicht ist. Die organische Phase wird abgetrennt, die wässrige Phase dreimal mit je 150 ml Dichlormethan extrahiert, die vereinigten organischen Phasen über Magnesiumsulfat getrocknet und zur Trockene eingeengt.
Ausbeute 29,4 g (95 % d. Th.) eines farblosen Feststoffes

| Elementaranalyse: | | | | |
|---|---|---|---|---|
| ber.: | C 39.41 | H 5.99 | N 18.60 | l 29.75 |
| gef.: | C 39.65 | H 6.04 | N 18.55 | I 29.63 |

### d) 2,4,6-Triiod-5-({10-[1,4,7-tris-(carboxymethyl)-1,4,7,10-tetraazacyclododecanyl]}acetyl)aminoisophthalsäure-N,N-bis-(3-aza-4-oxopentan-1,5-diyl-{10-[1,4,7-tris-(carboxymethyl)-1,4,7,10-tetraazacyclododecanyl]})amid

28 g (21,7 mmol) 2,4,6-Triiod-5-[(1,4,7,10-tetraazacydododecanyl)acetyl]aminoisophthalsäure-*N,N*-bis-[3-aza-4-oxopentan-1,5-diyl-(1,4,7,10-tetraazacyclododecanyl)]amid werden in 300 ml Wasser gelöst, 31,3 g (332,3 mmol) Chloressigsäure hinzugegeben und bei 60 °C mit 32 %iger NaOH ein pH-Wert von 9.5 eingestellt. Es wird 10 h auf 70 °C erhitzt, wobei man den pH-Wert der Reaktionsmischung kontinuierlich auf 9.5 nachstellt. Nach Abkühlen auf Raumtemp. wird mit konz. HCI ein pH-Wert von 1 eingestellt und die Lösung im Vakuum eingedampft. Der Rückstand wird mit 200 ml Methanol ausgerührt, von unlöslichen Bestandteilen abfiltriert und das Filtrat eingedampft. Der Rückstand wird in 300 ml Wasser gelöst und auf eine lonenaustauscher-Säule (1200 ml, IR 120, H⁺-form) gegeben. Anschließend wird mit 5 l Wasser gewaschen und das saure Eluat eingedampft. Der Rückstand wird in 150 ml Methanol gelöst und in 3000 ml Diethylether getropft, der dabei anfallende Feststoff abgesaugt, mit Diethylether mehrfach nachgewaschen und im Vakuum getrocknet.
Ausbeute 28,6 g (73 % d. Th.) eines farblosen Feststoffes

| Elementaranalyse: | | | | |
|---|---|---|---|---|
| ber.: | C 39.99 | H 5.26 | N 13.21 | I 20.42 |
| gef.: | C 40.21 | H 5.31 | N 13.19 | I 20.28 |

### e) 2,4,6-Triiod-5-((10-[1,4,7-tris-(carboxymethyl)-1,4,7,10-tetraazacyclododecanyl])acetyl, Gd-Komplex)aminoisophthalsäure-N,N-bis-(3-aza-4-oxopentan-1,5-diyl-{10-[1,4,7-tris-(carboxymethyl)-1,4,7,10-tetraazacyclododecanyl, Gd-Komplex]})amid

12,4 g (6,9 mmol) 2,4,6-Triiod-5-({10-[1,4,7-tris-(carboxymethyl)-1,4,7,10-tetraazacyclododecanyl]}acetyl)aminoisophthalsäure-*N*,*N* bis-(3-aza-4-oxopentan-1,5-diyl-{10-[1,4,7-tris-(carboxymethyl)-1,4,7,10-tetraazacyclododecanyl]})amid werden in 100 ml Wasser gelöst und durch Zugabe von 3 ml Essigsäure angesäuert. Es werden 3,7 g (10,4 mmol) Gadoliniumoxid zugegeben und 6 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 10/10/1). Die das Produkt enthaltenden Fraktionen werden vereinigt und mit 10 g lonenaustauscher (IR 267 H-Form) 2 h ausgerührt und abfiltriert, dann mit 10 g lonenaustauscher (IRA 67 OH-Form) 2 h ausgerührt, abfiltriert, mit 2 g Aktivkohle versetzt, 2 h auf 60 °C erwärmt, abfiltriert und gefriergetrocknet.
Ausbeute 8,1 g (48 % d. Th.) eines farblosen Feststoffes
Wassergehalt (Karl-Fischer): 6,9 %

| Elementaranalyse (bezogen auf die wasserfreie Substanz): | | | | | |
|---|---|---|---|---|---|
| ber.: | C 32.81 | H 3.78 | N 10.51 | I 16.81 | Gd 20.83 |
| gef.: | C 32.99 | H 3.81 | N 10.49 | I 16.75 | Gd 20.69 |

### Beispiel 8

### a) N,N-Bis-[2-tert-butoxycarbonylaminoacetyl-(2-aminoethyl)]-5-(methylamino)-2,4,6-triiodisophthalsäureamid

Eine Lösung von 10g (16,4 mmol) 2,4,6-Triiod-5-(methylamino)-isophthalsäuredichlorid (EP 0033426, Sovak, 1/80 US) in 100 ml Tetrahydrofuran wird zu einer Lösung von 7,82 g (36 mmol) [(2-Aminoethylcarbamoyl)methyl]carbaminsäure-*tert*-butylester (Sobirov et al., Russ. J. Bioorg. Chem. (Engl. Transl.) 1994, 397) und 10 ml Triethylamin in 200 ml Tetrahydrofuran über 1 h bei Raumtemp. getropft und 14 h nachgerührt. Es wird von unlöslichen Bestandteilen abfiltriert, zur Trockene eingeengt und der Rückstand an Kieselgel chromatographiert (Laufmittel Ethylacetat/Hexan 1 : 1). Die das Produkt enthaltenden Fraktionen werden vereinigt und eingedampft.
Ausbeute: 13,5 g (85 % d. Th.) eines farblosen Feststoffes

| Elementaranalyse: | | | | |
|---|---|---|---|---|
| ber.: | C 34.69 | H 4.37 | N 10.11 | l 39.27 |
| gef.: | C 24.91 | H 4.45 | N 10.98 | I 39.04 |

### b) N,N- Bis-[2-(2-aminoacetylamino)ethyl]-5-(methylamino)-2,4,6-triiodisophthalsäureamid

13 g (13,4 mmol) *N*,*N*-Bis-[2-*tert*-butoxycarbonylaminoacetyl-(2-aminoethyl)]-5-(methylamino)-2,4,6-triiodisophthalsäureamid werden bei 0-5°C vorsichtig mit 100 ml Trifluoressigsäure versetzt und 3 h bei Raumtemp. gerührt. Anschließend wird die Reaktionsmischung in 1500 ml Diethylether gegossen, der dabei anfallende Feststoff abgesaugt und mit Diethylether mehrfach nachgewaschen. Der Rückstand wird in 200 ml Wasser und 200 ml Dichlormethan gelöst unter starkem Rühren gibt man so lange 32proz. NaOH-Lösung bis ein pH-Wert von 10 erreicht ist. Die organische Phase wird abgetrennt, die wässrige Phase dreimal mit je 100 ml Dichlormethan extrahiert, die vereinigten organischen Phasen über Magnesiumsulfat getrocknet und zur Trockene eingeengt.
Ausbeute: 9,9 g (96 % d. Th.) eines farblosen Feststoffes

| Elementaranalyse: | | | | |
|---|---|---|---|---|
| ber.: | C 26.48 | H 3.14 | N 12.71 | I 49.37 |
| gef.: | C 26.64 | H 3.12 | N 12.76 | l 49.19 |

### c) N,N-Bis-[2-(2-brompropionylaminoacetylamino)ethyl)-5-[(2-brompropionyl)methylamino]-2,4,6-triiodisophthalsäureamid

57,8 g (75 mmol) *N,N*-Bis-[2-(2-aminoacetylamino)ethyl]-5-(methylamino)-2,4,6-triiodisophthalsäureamid werden in 500 ml Dimethylacetamid gelöst und bei 0 °C 75,5 g (350 mmol) 2-Brompropionsäurebromid (Aldrich) über 15 min zugetropft. Anschließend wird 20 h bei 40 °C gerührt. Die Reaktionsmischung wird in 4000 ml Eiswasser gegossen, der anfallende Feststoff abfiltriert, in 800 ml Ethylacetat gelöst und dreimal mit je 250 ml Wasser extrahiert. Die organische Phase wird über Natriumsulfat getrocknet, das Lösungsmittel zur Trockene eingeengt. Das Rohprodukt wird aus Methyl-*tert*-butylether umkristallisiert.
Ausbeute: 54,8 g (62 % d. Th.) eines farblosen Feststoffes

| Elementaranalyse: | | | |
|---|---|---|---|
| ber.: | C 26.55 | H 2.83 | N 8.34 |
| gef.: | C 26.77 | H 2.86 | N 8.21 |

### d) 2,4,6-Triiod-5-(2-{10-[1,4,7-tris-(benzyloxycarbonyl)-1,4,7,10-tetraazacyclododecanyl]}-propionyl)methylaminoisophthalsäure-N,N-bis-(3,6-diaza-4,7-dioxo-8-methyloctan-1,5-diyl-{10-[1,4,7-tris-(benzyloxycarbonyl)-1,4,7,10-tetraazacyclododecanyl]})amid

98,9 g (172,1 mmol) 1,4,7-Tris-(benzyloxycarbonyl)-1,4,7,10-tetrazacyclododecan (Delaney et al. , J. Chem. Soc. Perkin Trans. 1991, 3329) werden in 800 ml Acetonitril gelöst und mit 47,6 g (344,3 mmol) Natriumcarbonat versetzt. Anschließend werden unter starkem Rühren 54 g (45,9 mmol) *N,N*-Bis-[2-(2-brompropionylaminoacetylamino)ethyl)-5-[(2-brompropionyl)methylamino]-2,4,6-triiodisophthalsäureamid zugegeben und 20 h unter Rückfluß erhitzt. Es wird von unlöslichen Bestandteilen abfiltriert, zur Trockene eingeengt und der Rückstand an Kieselgel chromatographiert (Laufmittel Dichlormethan/Methanol 20:1). Die das Produkt enthaltenden Fraktionen werden vereinigt und eingedampft.
Ausbeute 52,6 g (43 % d. Th.) eines farblosen Feststoffes

| Elementaranalyse: | | | | |
|---|---|---|---|---|
| ber.: | C 55.14 | H 5.46 | N 10.01 | I 14.33 |
| gef.: | C 55.27 | H 5.44 | N 9.98 | I 14.21 |

### e) 2,4,6-Triiod-5-[2-(1,4,7,10-tetraazacyclododecanyl)propionyl]methylaminoisophthalsäure-N,N-bis-[3,6-diaza-4,7-dioxo-8-methytoctan-1,5-diyl-(1,4,7,10-tetraazacyclododecanyl)]amid

51,5 g (19,4 mmol) 2,4,6-Triiod-5-(2-{10-[1,4,7-tris-(benzyloxycarbonyl)-1,4,7,10-tetraazacyclododecanyl]}propionyl)methylaminoisophthalsäure-*N,N*-bis-(3,6-diaza-4,7-dioxo-8-methyloctan-1,5-diyl-{10-[1,4,7-tris-(benzyloxycarbonyl)-1,4,7,10-tetraazacyclododecanyl]})amid werden bei 0-5°C vorsichtig mit 500 ml HBr/AcOH (33%) versetzt und 3 h bei Raumtemp. gerührt. Anschließend wird die Reaktionsmischung in 2500 ml Diethylether gegossen, der dabei anfallende Feststoff abgesaugt und mit Diethylether mehrfach nachgewaschen. Der Rückstand wird in 300 ml Wasser und 300 ml Dichlormethan gelöst unter starkem Rühren gibt man so lange 32proz. NaOH-Lösung bis ein pH-Wert von 10 erreicht ist. Die organische Phase wird abgetrennt, die wässrige Phase dreimal mit je 150 ml Dichlormethan extrahiert, die vereinigten organischen Phasen über Magnesiumsulfat getrocknet und zur Trockene eingeengt.
Ausbeute 26,7 g (95 % d. Th.) eines farblosen Feststoffes

| Elementaranalyse: | | | | |
|---|---|---|---|---|
| ber.: | C 41.41 | H 6.26 | N 18.35 | I 26.25 |
| gef.: | C 41.57 | H 6.28 | N 17.29 | I 26.09 |

### f) 2,4,6-Triiod-5-(2-{10-[1,4,7-tris-(carboxymethyl)-1,4,7,10-tetraazacyclododecanyl]}propionyl)methylaminoisophthalsäure-N,N-bis-(3,6-diaza-4,7-dioxo-8-methyloctan-1 ,5-diyl-{1 0-[1 ,4,7-tris-(carboxymethyl)-1 ,4,7,10-tetraazacyclododecanyl]})amid

25,8 g (17,8 mmol) 2,4,6-Triiod-5-[2-(1,4,7,10-tetraazacyclododecanyl)-propionyl]methylaminoisophthalsäure-N,N-bis-[3,6-diaza-4,7-dioxo-8-methyloctan-1,5-diyl-(1,4,7,10-tetraazacyclododecanyl)]amid werden in 200 ml Wasser gelöst, 25,7 g (272,7 mmol) Chloressigsäure hinzugegeben und bei 60 °C mit 32 %iger NaOH ein pH-Wert von 9.5 eingestellt. Es wird 10 h auf 70 °C erhitzt, wobei man den pH-Wert der Reaktionsmischung kontinuierlich auf 9.5 nachstellt. Nach Abkühlen auf Raumtemp. wird mit konz. HCl ein pH-Wert von 1 eingestellt und die Lösung im Vakuum eingedampft. Der Rückstand wird mit 300 ml Methanol ausgerührt, von unlöslichen Bestandteilen abfiltriert und das Filtrat eingedampft. Der Rückstand wird in 200 ml Wasser gelöst und auf eine lonenaustauscher-Säule (1200 ml, IR 120, H⁺-form) gegeben. Anschließend wird mit 5 I Wasser gewaschen und das saure Eluat eingedampft. Der Rückstand wird in 150 ml Methanol gelöst und in 2500 ml Diethylether getropft, der dabei anfallende Feststoff abgesaugt, mit Diethylether mehrfach nachgewaschen und im Vakuum getrocknet.
Ausbeute 23,2 g (66 % d. Th.) eines farblosen Feststoffes

| Elementaranalyse: | | | | |
|---|---|---|---|---|
| ber.: | C41.41 | H 5.52 | N 13.49 | I 19.30 |
| gef.: | C 41.62 | H 5.53 | N 13.37 | I 19.13 |

### g) 2,4,6-Triiod-5-(2-{10-[1,4,7-tris-(carboxymethyl)-1,4,7,10-tetraazacyclododecanyl]}propionyl, Gd-Komplex)methylaminoisophthalsäure-N,N-bis-(3,6-diaza-4,7-dioxo-8-methyloctan-1,5-diyl-{10-[1,4,7-tris-(carboxymethyl)-1,4,7,10-tetraazacyclododecanyl, Gd-Komplex]})amid

13,6 g (6,9 mmol) 2,4,6-Triiod-5-(2-{10-[1,4,7-tris-(carboxymethyl)-1,4,7,10-tetraazacyclododecanyl]}propionyl)methylaminoisophthalsäure-N,N-bis-(3,6-diaza-4,7-dioxo-8-methyloctan-1,5-diyl-{10-[1,4,7-tris-(carboxymethyl)-1,4,7,10-tetraazacyclododecanyl]})amid werden in 100 ml Wasser gelöst und durch Zugabe von 3 ml Essigsäure angesäuert. Es werden 3,7 g (10,4 mmol) Gadoliniumoxid zugegeben und 6 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 10/10/1). Die das Produkt enthaltenden Fraktionen werden vereinigt und mit 10 g lonenaustauscher (IR 267 H-Form) 2 h ausgerührt und abfiltriert, dann mit 10 g lonenaustauscher (IRA 67 OH-Form) 2 h ausgerührt, abfiltriert, mit 2 g Aktivkohle versetzt, 2 h auf 60 °C erwärmt, abfiltriert und gefriergetrocknet.
Ausbeute 11,1 g (61 % d. Th.) eines farblosen Feststoffes
Wassergehalt (Karl-Fischer): 7,5 %

| Elementaranalyse (bezogen auf die wasserfreie Substanz): | | | | | |
|---|---|---|---|---|---|
| ber.: | C 33.54 | H 4.10 | N 10.93 | l 15.63 | Gd 19.37 |
| gef.: | C 33.71 | H 4.08 | N 10.88 | l 15.49 | Gd 19.21 |

### Beispiel 9

### Vergleichsversuch

Die im nächstliegendem Stand der Technik aus der US 5,660,814 offenbarten Verbindungen 3 aus Example 3 und 4 aus Example 4 sind im Vergleich zu den erfindungsgemäßen Verbindungen
5) ionisch und haben somit eine um Faktor 2 höhere Osmolalität im Vergleich zu den erfindungsgemäßen neutralen Verbindungen, was besonders negativ bei hohe Dosen ist,
6) diese sind wesentlich liphophiler als die erfindungsgemäßen Verbindungen (Anmerkung in Spalte 5/Zeile 29 der US 5 660 814, die Verbindungen aus dem Stand der Technik sind als Leberkontrastmittel verwendbar),
7) die Substanzen 3 und 4 der US 5 660 814 sind wesentlich toxischer als die erfindungsgemäßen Verbindungen (siehe LD₅₀ sowie Verteilungskoefficient) und
8) die Relaxivity für die Bildgebung im MR ist somit schlechter.

| | Beispiel 1 | Beispiel 5 | Beispiel 7 | Beispiel 8 | Beispiel 3 aus US 5,660,814 | Beispiel 4 aus US 5,660,814 A |
|---|---|---|---|---|---|---|
| Osmolalitat 200mg(I+Gd )/ml [mosmol/l] | 358 | 371 | 334 | 346 | ~ 750 als Na-Salz | ~ 730 als Na-Salz |
| Verteilungsk oefficient BuOH/H₂O | 0.00005 | 0.00006 | 0.00005 | 0.00005 | 0.007 | 0.01 |
| Relaxivity T1 in H₂O | 9.8 | 10.1 | 10.3 | 10.0 | 6.3 | 7.2 |
| LD₅₀ (Maus) | >12.0 g(l + Gd)/kg | >12.0 g(l + Gd)/kg | >12.0 g(l + Gd)/kg | >12.0 g(l + Gd)/kg | >2.0 g(l + Gd)/kg | >2.0 g(l + Gd)/kg |

## Patentansprüche

1. Metallkomplexe der allgemeinen Formel I 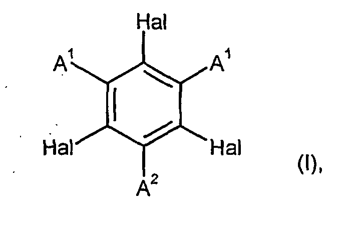 worin
Hal für Brom oder Jod,
A¹ für den Rest
-CONR¹-(CH₂)ₙ-NR²-(CO-CHZ¹-NH)ₘ-CO-CHZ²-K,
A² für den Rest -NR¹-CO-CHZ²-K stehen,
in denen R¹ und R² unabhängig ein Wasserstoffatom, eine C₁-C₂-Alkylgruppe oder eine Monohydroxy-C₁-C₂-Alkylgruppe,
Z¹ und Z² unabhängig voneinander ein Wasserstoffatom oder eine Methylgruppe,
n die Ziffern 2-4,
m die Ziffern 0 oder 1 und
K für einen Makrocyclus der Formel I_{A} 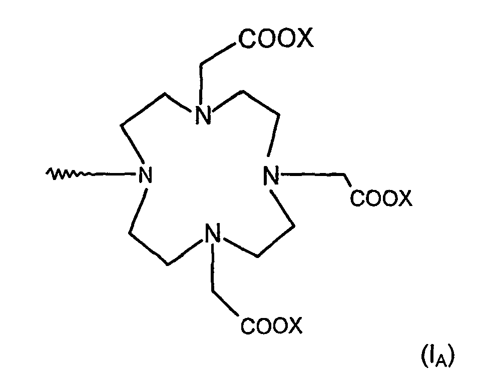 mit X in der Bedeutung eines Wasserstoffatoms oder eines Metallionenäquivalents der Ordnungszahlen 20-29,39, 42, 44 oder 57-83 stehen, mit den Maßgaben, dass mindestens zwei X für Metallionenäquivalente stehen und gegebenenfalls vorhandene freie Carboxygruppen gegebenenfalls als Salze organischer und/oder anorganischer Basen oder Aminosäuren oder Aminosäureamide vorliegen.

2. Metallkomplexe nach Anspruch 1, **dadurch gekennzeichnet, dass** A¹ für eine
-CONH(CH₂)_{2;3}NHCOCH₂NHCOCH(CH₃)-K,
-CONH(CH₂)_{2;3}NHCOCH₂NHCOCH₂-K,
-CONH(CH₂)_{2;3}NHCOCH₂-K,
-CONH(CH₂)_{2;3}NHCOCH(CH₃)-K,
-CON(CH₂CH₂OH(CH₂)₂NHCOCH₂-K
Gruppe steht.

3. Metallkomplexe nach Anspruch 1, **dadurch gekennzeichnet, dass** A² für eine
-NHCOCH(CH₃)-K,
-NHCOCH₂-K,
-N(CH₃)COCH₂-K,
-N(CH₃)COCH(CH)₃-K,
-N(CH₂CH₂OH)COCH₂-K,
-N(CH₂CH₂OH)COCH(CH₃)-K
Gruppe steht.

4. Metallkomplexe nach Anspruch 1, **dadurch gekennzeichnet, dass** X für ein Metallionenäquivalent der Ordnungszahlen 21-29, 42, 44, 58-70 steht.

5. Metallkomplexe nach Anspruch 4, **dadurch gekennzeichnet, dass** X für ein Metallionenäquivalent der Ionen Gadolinium (III), Dysprosium (III), Europium (III), Eisen(III) oder Mangan (II) steht.

6. Pharmazeutische Mittel enthaltend mindestens einen Metallkomplex der allgemeinen Formel I gemäß Anspruch 1, gegebenenfalls mit den in der Galenik üblichen Zusätzen.

7. Verwendung von mindestens einem Metallkomplex nach Anspruch 1 für die Herstellung von Mitteln für die Röntgen-Diagnostik.

8. Verwendung von mindestens einem Metallkomplex nach Anspruch 4 für die Herstellung von Mitteln für die MRT-Diagnostik.

9. Pharmazeutische Mittel enthaltend je einen Metallkomplex nach Anspruch 1 und 4 in einem Molverhältnis von 2000:1 bis 1:1, bevorzugt 49:1 bis 4:1.

10. Pharmazeutische Mittel nach Anspruch 6, **dadurch gekennzeichnet, dass** der/die in Wasser oder physiologischer Salzlösung gelöste(n) oder suspendierte(n) Metallkomplex(e) in einer Konzentration von 0,001 bis 1 Mol/l vorliegt/vorliegen.

11. Verwendung von mindestens einem Metallkomplex nach Anspruch 1 für die Herstellung von Mitteln für die Röntgen- und MR Diagnostik von Gehirninfarkten und Tumoren der Leber bzw. raumfordernden Prozesse in der Leber sowie von Tumoren des Abdomens (inklusive der Nieren) und des Muskel-Skelett-Systems und für die Darstellung von Blutgefäßen nach intraarterieller oder intravenöser Injektion.

12. Verfahren zur Herstellung der Metallkomplexe der allgemeinen Formel I gemäß Anspruch 1, **dadurch gekennzeichnet, dass** man einen Trijod- oder Tribromaromaten der allgemeinen Formel II 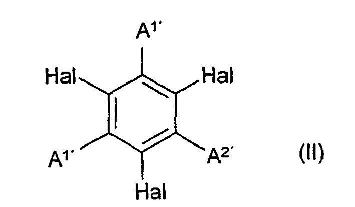 in an sich bekannter Weise mit einem Makrocyclus der allgemeinen Formel III 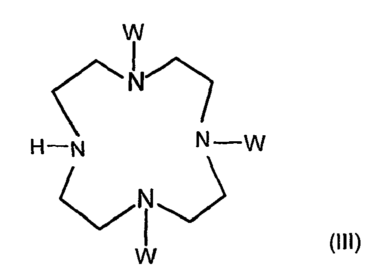 worin
W für eine Schutzgruppe oder eine -CH₂COOX'-gruppe mit X' in der Bedeutung von X oder einer Schutzgruppe und A^{1'} in der Bedeutung von - CO-NR¹-(CH₂)ₙ-NR²-(CO-CHZ¹-NH)ₘ-CO-CHZ²-Hal' und A^{2'} in der Bedeutung von -NR¹-CO-CHZ²-Hal' mit Hal' in der Bedeutung von Chlor oder Brom stehen, umsetzt und anschließend gegebenenfalls die Schutzgruppe W entfernt und die Reste CH₂COOX in an sich bekannter Weise einführt bzw. die für X' gegebenenfalls stehende Schutzgruppe entfernt und anschließend in an sich bekannter Weise mit einem Metalloxid oder Metallsalz eines Elements der Ordnungszahlen 20-29, 39, 42, 44 oder 57-83 umsetzt.

13. Verfahren zur Herstellung der pharmazeutischen Mittel gemäß Anspruch 6, **dadurch gekennzeichnet, dass** man die in Wasser oder physiologischer Salzlösung gelöste oder suspendierte Komplexverbindung, gegebenenfalls mit den in der Galenik üblichen Zusätzen, in eine für die enterale oder parenterale Applikation geeignete Form bringt.

## Claims

1. Metal complexes of general formula I 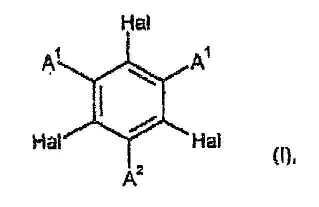 in which
Hal stands for bromine or iodine,
A¹ stands for the radical
-CONR¹-(CH₂)ₙ-NR²-(CO-CHZ¹-NH)ₘ-CO-CHZ²-K,
A² stands for the radical -NR¹-CO-CHZ²-K,
in which R¹ and R², independently, are a hydrogen atom, a C₁-C₂-alkyl group or a monohydroxy-C₁-C₂-alkyl group,
Z¹ and Z², independently of one another, are a hydrogen atom or a methyl group,
n is the numbers 2-4,
m is the number 0 or 1, and
K stands for a macrocyclic compound of formula I_{A} 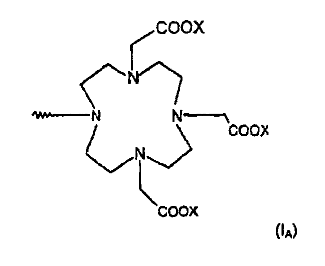 with X in the meaning of a hydrogen atom or a metal ion equivalent of atomic numbers 20-29, 39, 42, 44 or 57-83, provided that at least two X stand for metal ion equivalents and optionally present free carboxy groups optionally are present as salts of organic and/or inorganic bases or amino acids or amino acid amides.

2. Metal complexes according to Claim 1, **characterized in that** A¹ stands for a
-CONH(CH₂)_{2;3}NHCOCH₂NHCOCH(CH₃)-K,
-CONH(CH₂)_{2;3}NHCOCH₂NHCOCH₂-K,
-CONH (CH₂)_{2; 3}NHCOCH₂-K,
-CONH(CH₂)_{2;3}NHCOCH(CH₃)-K, or
-CON (CH₂CH₂OH (CH₂) ₂NHCOCH₂-K group .

3. Metal complexes according to Claim 1, **characterized in that** A² stands for a
-NHCOCH(CH₃)-K,
-NHCOCH₂-K,
-N (CH₃) COCH₂-K,
-N(CH₃)COCH(CH)₃-K,
-N (CH₂CH₂OH) COCH₂-K, or
-N (CH₂CH₂OH)COCH(CH₃)-K group.

4. Metal complexes according to Claim 1, **characterized in that** X stands for a metal ion equivalent of atomic numbers 21-29, 42, 44 or 58-70.

5. Metal complexes according to Claim 4, **characterized in that** X stands for a metal ion equivalent of ions gadolinium (III), dysprosium (III), europium(III), iron(III) or manganese (II).

6. Pharmaceutical agents that contain at least one metal complex of general formula I according to Claim 1, optionally with the additives that are commonly used in galenicals.

7. Use of at least one metal complex according to Claim 1 for the production of agents for x-ray diagnosis.

8. Use of at least one metal complex according to Claim 4 for the production of agents for MRT diagnosis.

9. Pharmaceutical agents that each contain a metal complex according to Claim 1 and 4 in a molar ratio of 2000:1 to 1:1, preferably 49:1 to 4:1.

10. Pharmaceutical agents according to Claim 6, **characterized in that** the metal complex(es) dissolved or suspended in water or physiological salt solution is (are) present at a concentration of 0.001 to 1 mol/l.

11. Use of at least one metal complex according to Claim 1 for the production of agents for x-ray and MR diagnosis of cerebral infarctions and tumors of the liver or space-occupying processes in the liver as well as tumors of the abdomen (including the kidneys) and the muscle-skeleton system and for the visualization of blood vessels after intraarterial injection or intravenous injection.

12. Process for the production of metal complexes of general formula I according to Claim 1, **characterized in that** a triiodoaromatic compound or a tribromoaromatic compound of general formula II 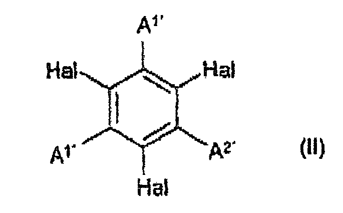 is reacted in a conventional manner with a macrocyclic compound of general formula III 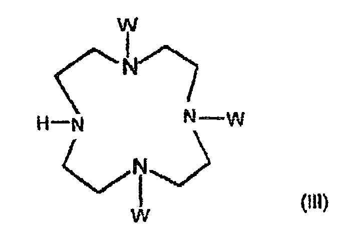 in which W stands for a protective group or a -CH₂COOX' group with X' in the meaning of X or a protective group, and A^{1'} in the meaning of -CO-NR¹- (CH₂) ₙ-NR²- (CO-CHZ¹-NH)ₘ-CO-CHZ²-Hal' and A^{2'} in the meaning of -NR¹-CO-CHZ²-Hal' with Hal' in the meaning of chlorine or bromine, and then optionally protective group W is removed and the radicals CH₂COOX are introduced in a way that is known per se or the protective group that optionally stands for X' is removed and then reacted in a way that is known per se with a metal oxide or metal salt of an element of atomic numbers 20-29, 39, 42, 44 or 57-83.

13. Process for the production of pharmaceutical agents according to Claim 6, **characterized in that** the complex compound that is dissolved or suspended in water or physiological salt solution, optionally with the additives that are commonly used in galenicals, is brought into a form that is suitable for enteral or parenteral administration.

## Revendications

1. Complexes métalliques de formule générale I 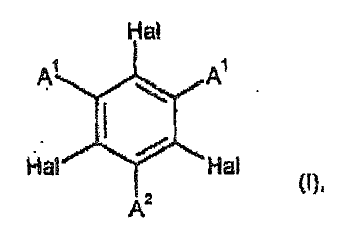 dans laquelle
Hal signifie le brome ou l'iode,
A¹ représente le radical
-CONR¹-(CH₂)ₙ-NR²-(CO-CHZ¹-NH)ₘ-CO-CHZ²-K
A² représente le radical -NR¹-CO-CHZ²-K,
radicaux dans lesquels R¹ et R² représentent indépendamment un atome d'hydrogène, un groupe alkyle en C₁-C₂ ou un groupe monohydroxy-alkyle en C₁-C₂,
Z¹ et Z² représentent, indépendamment l'un de l'autre, un atome d'hydrogène ou un groupe méthyle,
n représente les chiffres 2-4,
m représente le chiffre 0 ou 1 et
K représente un macrocycle de formule I_{A} 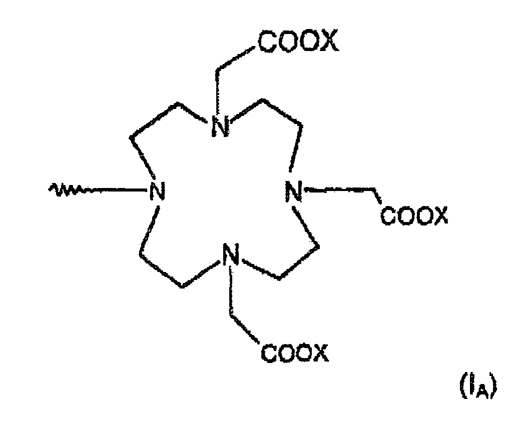 où X a la signification d'un atome d'hydrogène ou d'un équivalent d'ion métallique des nombres atomiques 20-29, 39, 42, 44 ou 57-83, étant entendu qu'au moins deux X représentent des équivalents d'ions métalliques et que les groupes carboxy libres éventuellement présents se trouvent éventuellement sous forme de sels avec des bases organiques et/ou minérales ou avec des acides aminés ou des amides d'acides aminés.

2. Complexes métalliques selon la revendication 1, **caractérisés en ce que** A¹ représente un groupe
-CONH (CH₂)_{2;3}NHCOCH₂NHCOCH (CH₃)-K,
-CONH(CH₂)_{2;3}NHCOCH₂NHCOCH₂-K,
-CONH(CH₂)_{2;3}NHCOCH₂-K,
-CONH (CH₂)_{2;3}NHCOCH (CH₃) -K,
-CON(CH₂CH₂OH(CH₂)₂NHCOCH₂-K.

3. Complexes métalliques selon la revendication 1, **caractérisés en ce que** A² représente un groupe
-NHCOCH(CH₃)-K,
-NHCOCH₂-K,
-N(CH₃)COCH₂-K,
-N(CH₃)COCH(CH)₃-K,
-N(CH₂CH₂OH)COCH₂-K,
-N(CH₂CH₂OH)COCH(CH₃)-K.

4. Complexes métalliques selon la revendication 1, **caractérisés en ce que** X représente un équivalent d'ion métallique des nombres atomiques 21-29, 42, 44, 58-70.

5. Complexes métalliques selon la revendication 4, **caractérisés en ce que** X représente un équivalent d'ion métallique des ions gadolinium-(III), dysprosium-(III), europium-(III), fer-(III) ou manganèse-(II).

6. Composition pharmaceutique contenant au moins un complexe métallique de formule générale I selon la revendication 1, éventuellement avec les additifs usuels en galénique.

7. Utilisation d'au moins un complexe métallique selon la revendication 1, pour la préparation d'agents pour le diagnostic radiologique.

8. Utilisation d'au moins un complexe métallique selon la revendication 4, pour la préparation d'agents pour le diagnostic par MRT (tomographie par résonance magnétique).

9. Composition pharmaceutique contenant un complexe métallique selon la revendication 1 et un complexe métallique selon la revendication 4 en un rapport molaire allant de 2000:1 à 1:1, de préférence de 49:1 à 4:1.

10. Composition pharmaceutique selon la revendication 6, **caractérisée en ce que** le/les complexe(s) métallique(s) dissous ou en suspension dans de l'eau ou du sérum physiologique est/sont présent(s) à une concentration de 0,001 à 1 mole/l.

11. Utilisation d'au moins un complexe métallique selon la revendication 1, pour la préparation d'agents pour le diagnostic radiographique et par RMN d'infarctus cérébraux et de tumeurs du foie ou de processus expansifs dans le foie ainsi que de tumeurs de l'abdomen (y compris des reins) et du système musculaire-squelettique et pour la représentation des vaisseaux sanguins après injection intra-artérielle ou intraveineuse.

12. Procédé pour la préparation des complexes métalliques de formule générale I selon la revendication 1, **caractérisé en ce qu'**on fait réagir d'une façon connue en soi un composé aromatique tri-iodé ou tribromé de formule générale II 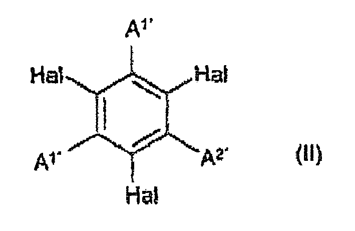 avec un macrocycle de formule générale III 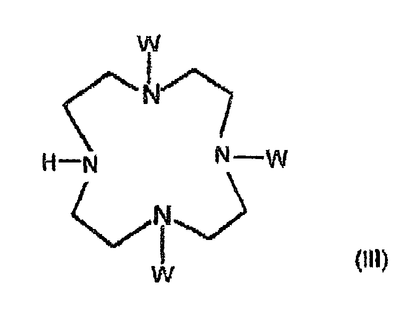 formules dans lesquelles
W représente un groupe protecteur ou un groupe -CH₂COOX' où X' a la signification de X ou d'un groupe protecteur et A^{1'} a la signification de CO-NR¹- (CH₂)ₙ-NR² (CO-CHZ¹-NH)ₘ-CO-CHZ²-Hal' et A^{2'} a la signification de -NR¹-CO-CHZ²-Hal' où Hal' signifie le chlore ou le brome, et ensuite éventuellement on élimine le groupe protecteur W et d'une façon connue en soi on introduit les radicaux CH₂COOX ou on élimine le groupe protecteur éventuellement présent pour X' et ensuite on fait réagir d'une façon connue en soi avec un oxyde métallique ou un sel métallique d'un élément des nombres atomiques 20-29, 39, 42, 44 ou 57-83.

13. Procédé pour la préparation d'une composition pharmaceutique selon la revendication 6, **caractérisé en ce que** l'on met sous une forme appropriée à l'administration entérale ou parentérale le composé complexe dissous ou en suspension dans de l'eau ou du sérum physiologique, éventuellement avec les additifs usuels en galénique.
